# EUROPEAN PATENT SPECIFICATION

(11) **EP 1 141 026 B1**
(45) Date of publication and mention of the grant of the patent: **29.11.2006**
(21) Application number: 99966605.0
(22) Date of filing: 22.12.1999
(51) Int. Cl.: C07K 16/00, C07K 16/28, C12N 5/20, G01N 33/68, G01N 33/577, A61K 39/39

(54) **COMPOSITIONS AND METHODS FOR GENERATING MONOCLONAL ANTIBODIES REPRESENTATIVE OF A SPECIFIC CELL TYPE**
ZUSAMMENSETZUNGEN UND VERFAHREN ZUR HERSTELLUNG MONOKLONALER ANTIKÖRPER, REPRÄSENTATIV FÜR EINEN SPEZIFISCHEN ZELLTYP
COMPOSITIONS ET PROCEDES PERMETTANT DE PRODUIRE DES ANTICORPS MONOCLONAUX REPRESENTATIFS D'UN TYPE DE CELLULE SPECIFIQUE

(30) Priority: 22.12.1998 US 218539
(43) Date of publication of application: 10.10.2001
(73) Proprietor: Raven Biotechnologies, Inc., South San Francisco, CA 94080 (US)
(72) Inventor: Mather, Jennie P., Millbrae, CA 94030 (US); Bald, Laura N., Sunnyvale, CA 94086 (US); Roberts, Penelope R., Millbrae, CA 94030 (US); Stephan, Jean-Philippe F., Millbrae, CA 94030 (US)
(74) Representative: Sexton, Jane Helen
(86) International application number: PCT/US1999/030741
(87) International publication number: WO 2000/037503

(56) References cited:
- EP-A- 0 856 520
- STEPHAN JEAN-PHILIPPE ET AL: "Characterization of cell surface proteins using antibodies raised to antigens from pancreatic cell lines." 37TH ANNUAL MEETING OF THE AMERICAN SOCIETY FOR CELL BIOLOGY;WASHINGTON, D.C., USA; DECEMBER 13-17, 1997, vol. 8, no. SUPPL., November 1997 (1997-11), page 328A XP000906996 Molecular Biology of the Cell Nov., 1997 ISSN: 1059-1524
- OKABE T ET AL: "MONOCLONAL ANTIBODIES TO SURFACE ANTIGENS OF SMALL CELL CARCINOMA OF THE LUNG" CANCER RESEARCH 1984, vol. 44, no. 11, 1984, pages 5273-5278, XP000906947 ISSN: 0008-5472
- DOTSIKAS G ET AL: "CELLULAR HETEROGENEITY IN NORMAL AND NEOPLASTIC HUMAN UROTHELIUM A STUDY USING MURINE MONOCLONAL ANTIBODIES" BRITISH JOURNAL OF CANCER 1987, vol. 56, no. 4, 1987, pages 439-444, XP000906997 ISSN: 0007-0920
- DATABASE MEDLINE [Online] US NATIONAL LIBRARY OF MEDICINE (NLM), BETHESDA, MD, US STEPHAN J P ET AL: "Distribution and function of the adhesion molecule BEN during rat development." retrieved from STN Database accession no. 1999365244 XP002137515 & DEVELOPMENTAL BIOLOGY, (1999 AUG 15) 212 (2) 264-77. ,

## Description

### TECHNICAL FIELD

This invention is in the field of immunology. Specifically, the invention relates to the generation of a population of monoclonal antibodies capable of binding to antigens, especially cell surface antigens, that are representative of a particular cell type. The compositions and methods embodied in the present invention are particularly useful for isolating monoclonal antibodies that are tissue-selective, sub-tissue selective or cell-type specific.

### BACKGROUND OF THE INVENTION

Cell surface antigens (CSAs) are molecules anchored on the cell plasma membrane. CSAs constitute a large family of proteins, glycoproteins, polysaccharides and lipids, which serve not only as structural constituents of the plasma membrane, but more importantly as regulatory elements governing a variety of biological functions. Numerous CSAs have been identified, cloned and found to play a pivotal role in the transduction of signals triggered by external stimuli such as growth factors and hormones that culminate in a wide range of cellular responses. Among them are cell division, differentiation, apoptosis, and motility. Defects in cell surface antigens, such as receptors and adhesion proteins in particular, are now known to account for a vast number of diseases, including numerous forms of cancer, vascular diseases and neuronal diseases.

The identification of cell surface antigens of a specific cell type often proceeds with the generation of monoclonal antibodies reactive with antigens present in that type of cells, followed by immunoaffinity purification of the surface antigens using the corresponding monoclonal antibodies. Traditional immunogens used for generating pools of monoclonal antibodies consist of membrane extracts or intact cells of a particular cell type that have been propagated in a serum-supplemented medium, although an abstract reporting the generation of mouse monoclonal antibodies reacting with cell surface proteins of rat ductal epithelial cell lines cultured in a serum-free system has been published (Stephen *et al.* 37^{th} Annual Meeting of the American Society for Cell Biology; Washington D.C. USA; December 13-17, 1997, vol. 8, no. SUPPL., November 1997 page 328a). Immunogens are generally administered in Freund's adjuvant as described in *Dotsikas et al.* (1987) Br. J. Cancer, 56: 439-444. Neither type of immunogen necessarily yields a population of monoclonal antibodies that specifically binds to antigens representative of a particular cell type; nor has it been optimized to produce monoclonal antibodies that bind to antigens in their native configurations. For example, Dotsikas *et al.* describes the production of three antibodies of which one was not cell-type specific. Extraction of the surface antigens involves detergents or other organic solvents that are known to dissemble the plasma membrane bilayer and thus dissociate the surface antigens from their native environment. The supplementation of serum in culturing cells to be used as a immunogen also has pronounced disadvantages.

Serum is an extremely complex mixture of many small and large biomolecules with undefined activities. For most cells, serum is not the physiological fluid which they contact in the original tissue from which they are derived. *In vivo,* a cell would be exposed to the equivalent of serum only under special circumstances involving tissue injury and blood coagulation. *In vitro,* various hormones and growth factors present in the serum can stimulate excessive growth and/or terminal differentiation, accompanied by an altered expression of cell surface antigens, secretory, cytosolic or nuclear proteins. The complex mixture of serum factors may also inhibit growth and/or differentiation of a particular cell type, resulting in a change in cell morphology and viability. Moreover, numerous kinds of serum biomolecules are known to adhere to the cell surfaces. These biomolecules include but are not limited to transfer proteins (e.g. albumin), attachment and spreading factors (e.g. collagen and fibronectin), and various kinds of serum lipids. Adsorption of these exogenous molecules to the cell surface not only results in the generation of antibodies cross-reacting with molecules unrepresentative of the specific cell type, but can also mask presentation of the native antigens, and thus further undermines the "representativeness" of the resulting monoclonal antibody pool.

Thus, there remains a considerable need for compositions and methods applicable for generating a population of monoclonal antibodies that specifically binds to antigens representative of a particular cell type. The production of these monoclonal antibodies would greatly facilitate the identification of novel antigens, and the delineation of the combination of surface antigens present on a specific cell type. The present invention satisfies these needs and provides related advantages as well.

### SUMMARY OF THE INVENTION

A principal aspect of the present invention is the design of a technique for generating a population of monoclonal antibodies capable of binding to antigens representative of a particular cell type. This technique of antibody production minimizes the generation of non-representative antibodies cross-reacting with proteins not present in a particular cell type. This technique also maximizes the preservation of intact antigens, especially surface antigens, for production of a plurality of monoclonal antibodies that bind to the native antigens of a particular cell type. Such method generates a unique pool of candidate antibodies that recognize antigens selectively expressed in certain body tissues (tissue-selective), localized to a specific region (sub-tissue selective) or a particular cell layer within those tissues (cell-type specific).

Accordingly, the present invention provides the use of viable and intact cells of a specific cell type, wherein the surfaces of the cells are free of serum and the cells are not human embryonic cells for immunizing a non-human host mammal to produce a population of monoclonal antibodies that bind to antigens representative of said cell type that are heterologous to the non-human host mammal, said immunizing comprising introducing into the mammal a plurality of said cells without adjuvant or with Ribi adjuvant.

In one aspect, the cells used for immunizing a host mammal have been cultured in a serum-free medium. In another aspect, the cells used for immunization have been grown in the form of a monolayer or aggregates. In yet another aspect, the cells have been grown on a biological or a non-biological substrate, wherein the biological substrate is selected from collagen, fibronectin, laminin and poly-lysine; and wherein the non-biological substrate is selected from nitrocellulose, nylon and polytetrafluoroethylene membrane.

In still another aspect, the cells used for immunization are fetal or adult cells. In still another aspect, the cells are of ectodermal, endodermal or mesodermal origin. In a preferred embodiment, the cells are selected from ASC, ESC, ROG, BUD, RED, NODD, BR516, RL-65 and NEP cells.

The present invention also provides a method of generating monoclonal antibodies binding to the surface antigens of a specific cell type. The method involves (a) fusing an immortalized cell line with lymphoid cells which have been taken from a mammal which has been immunized with a plurality of viable and intact cells of a specific cell type that are heterologous to the host mammal, wherein the surfaces of the cells were free of serum and the cells were introduced into the mammal without adjuvant or with Ribi adjuvant, to produce hybridomas that produce monoclonal antibodies; (b) culturing the hybridomas under the conditions favorable for the secretion of monoclonal antibodies; and (c) selecting the hybridomas that secrete monoclonal antibodies binding to surface antigens present on the viable and intact cells used for immunization. In one aspect, the selection of hybridomas is effected by an immunoassay, such as ELISA or immunoblotting. In one aspect, the selection of hybridomas is effected by a cell sorting process, e.g. FACS.

The present description includes populations of hybridomas and populations of monoclonal antibodies generated by the aforementioned method. In one aspect, the monoclonal antibodies so produced specifically bind to the extracellular domain of the cell surface antigens.

The present invention further provides a method of determining the combination of cell surface antigens present on a specific cell type, comprising the steps of: (a) fusing an immortalized cell line with lymphoid cells which have been taken from a mammal which has been immunized with a plurality of viable and intact cells of a specific cell type that is heterologous to the host mammal, wherein the surfaces of the cells were free of serum and the cells were introduced into the mammal without adjuvant or with Ribi adjuvant, to produce hybridomas that produce monoclonal antibodies; (b) culturing the hybridomas under the conditions favorable for the secretion of monoclonal antibodies; (c) selecting the hybridomas that produce monoclonal antibodies binding to the cell surface antigens present on the viable and intact cells of step (a); and (d) identifying the antigens to which the monoclonal antibodies bind, and thereby determining the combination of cell surface antigens present on said specific cell type.

In one aspect, identification of the corresponding antigens further involves obtaining cDNAs of the specific cell type, expressing the cDNAs in a second cell type at a level of at least 5 fold higher than that of the corresponding endogenous antigens, if present, and screening cells of the second cell type for a specific binding to the monoclonal antibodies produced by the hybridomas selected in (c) above.

### BRIEF DESCRIPTION OF THE DRAWINGS

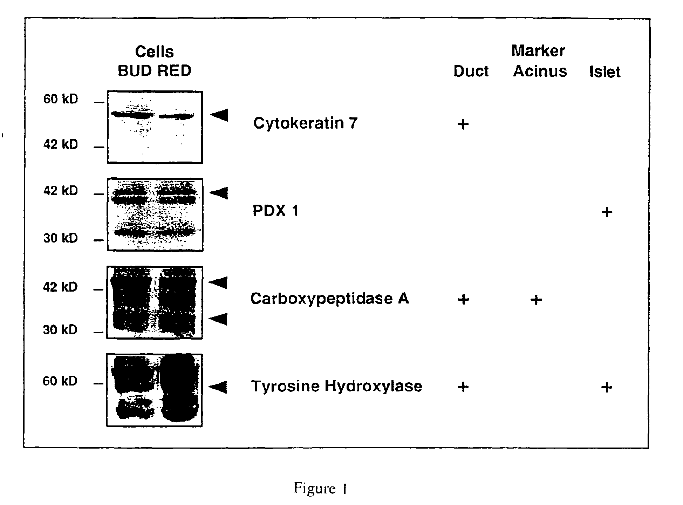

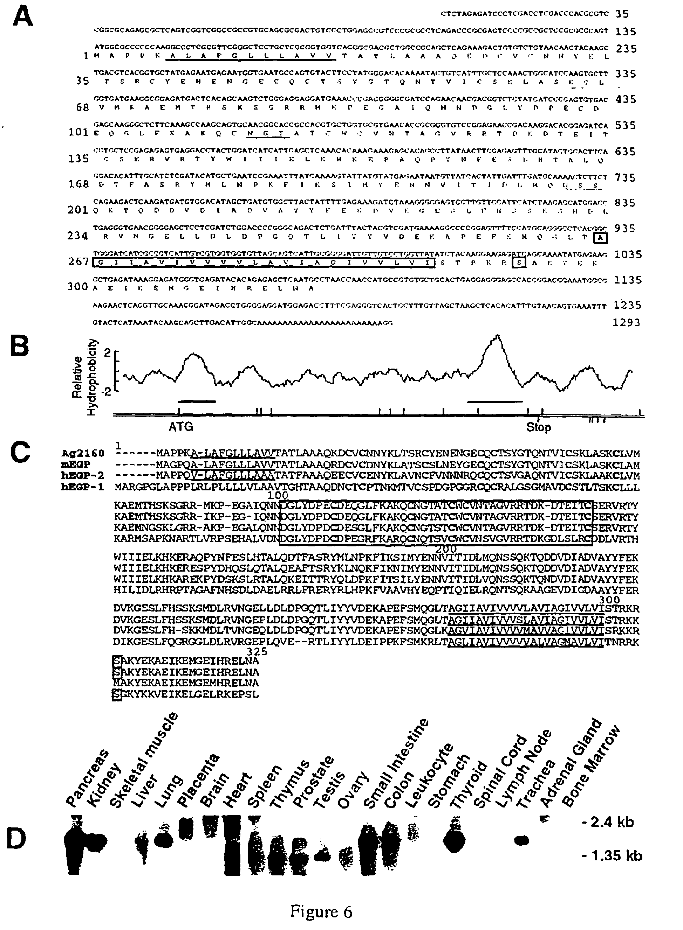

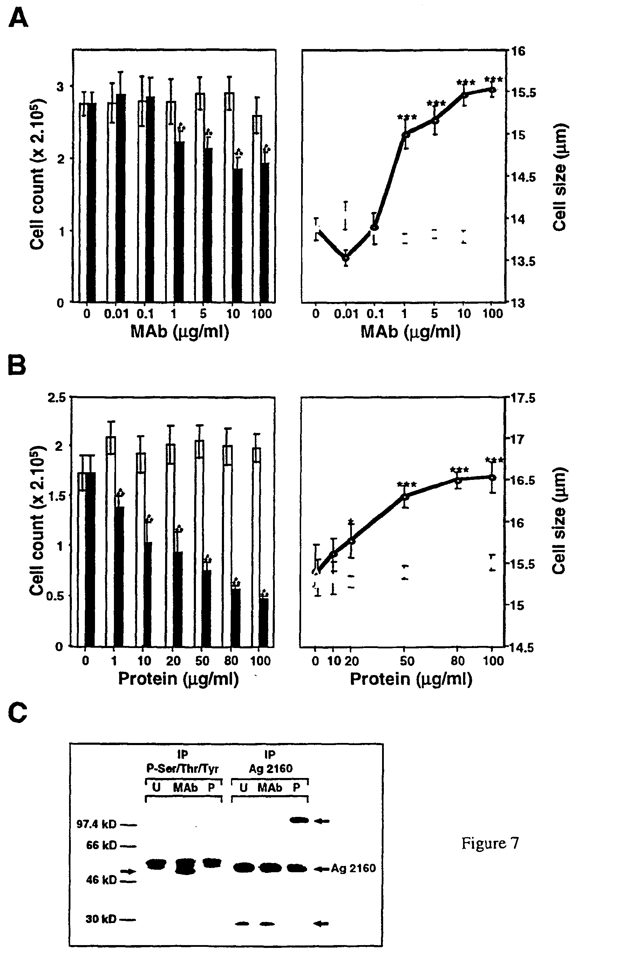

Figure 1 is an immunoblot of cell lysates prepared from the BUD and RED cells using antibodies directed to proteins known to be expressed at the early stage of pancreatic development. Approximately 100 µg of proteins were resolved by on a 4-20% SDS-polyacrylamide gradient gel, and immunoblotted with mouse monoclonal anti-human cytokeratin 7 (1/500), rabbit polyclonal anti-rat PDX1 (1/500), rabbit polyclonal anti-bovine carboxypeptidase A (1/500) or rabbit polyclonal anti-rat tyrosine hydroxylase (1/1000).

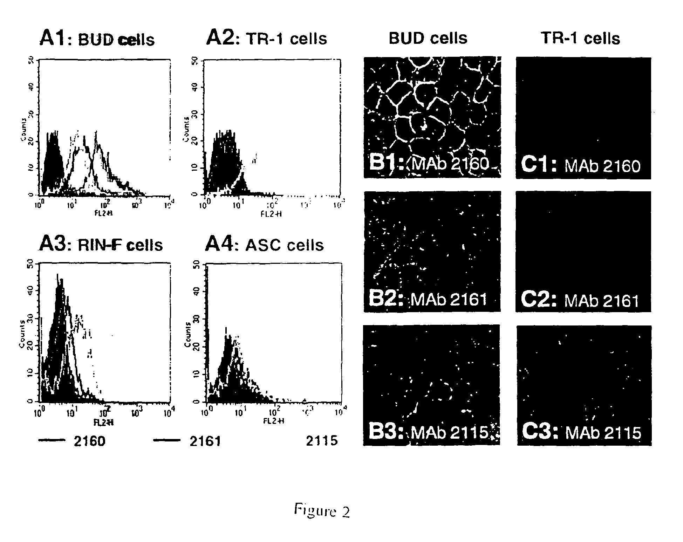

Figure 2 (A) depicts the results of FACS analysis of BUD, TR-1, RIN-F and ASC cells using monoclonal antibodies (MAbs) 2160 (red), 2161 (blue) and 2115 (orange). Controls (green) have no second antibody. Also shown is the immunofluorescence stain of BUD (B) and TR-1 (C) cells with MAbs 2160, 2161 and 2115.

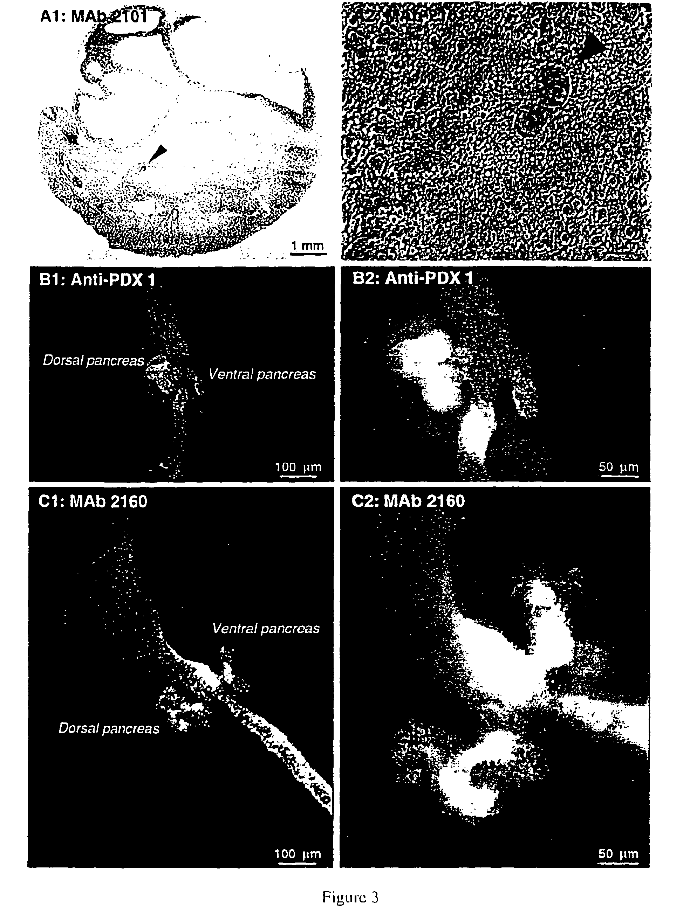

Figure 3 depicts immunolocalization of Ag 2101, PDX1 and Ag 2160 along the rat embryo gut. 12-Day rat embryo frozen section was stained with MAb 2101. Arrows indicate staining in the pancreatic bud (A1-2). Also shown is the stain of dissected 12.5-day rat embryo viscera with a rabbit polyclonal anti-rat PDX1 (B1-2) and MAb 2160 (C1-2).

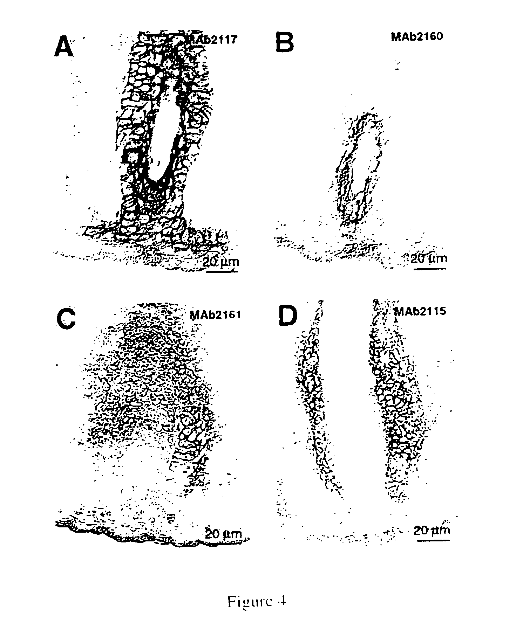

Figure 4 depicts staining of frozen sections from 18-day rat embryo vibrissa with MAbs 2117 (A), 2160 (B), 2161 (C) and 2115 (D). Note that even though this structure is recognized by all 4 antibodies, each monoclonal antibody stains a different subset of cells within the vibrissa.

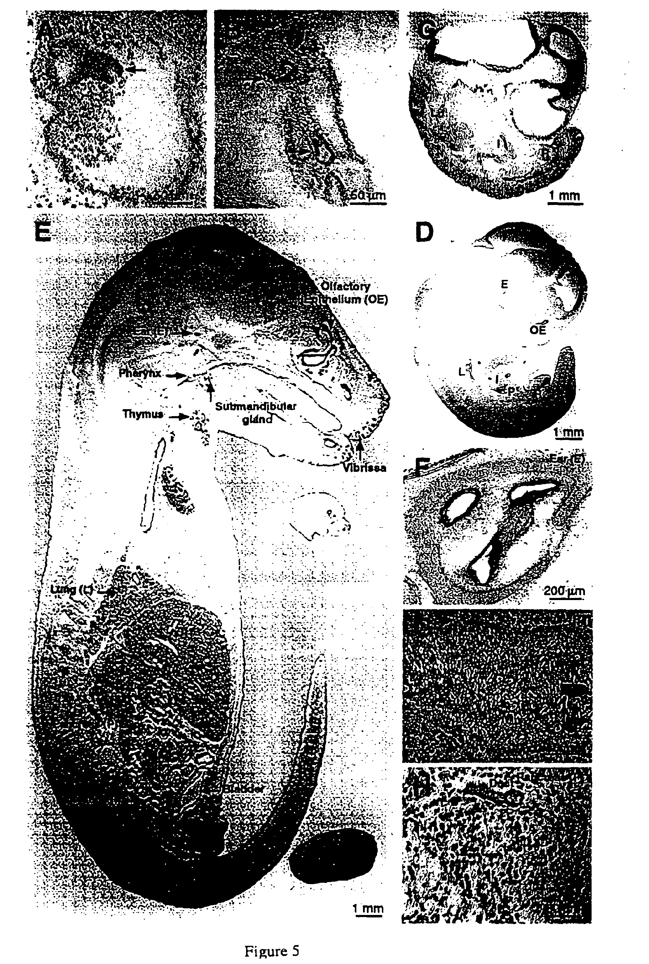

Figure 5 (A-E) depicts staining of frozen sections from rat embryos of day 9, 10, 12, 15 or 18 using MAb 2160. Mab 2160 stain was observed in epithelial cells in the vibrissa, olfactory epithelium (OE), ear (E), submandibular gland, pharynx, lung (L), pancreas (P), intestine (I), bladder, and rectum (R). Higher magnification reveals staining of e20 rat embryo ear (F), rectum (e18) (G), and adult pancreas (H). In (H), Mab 2160 stain was detected in the ductal epithelial cell but not in the islet cells.
Panel A of Figure 6 depicts the complete DNA sequence and the deduced amino acid sequence of Ag 2160. The nucleotide numbering is shown on the right and amino acid numbering is shown on the left of the sequence. The predicted sequence reveals a possible signal peptide (black overline), 2 potential N-linked glycosylation sites (gray overline), and a single 23-amino acid transmembrane domain (gray frame). Panel B is a Kyte-Doolittle plot of the deduced amino acid sequence. The predicted start and stop codons are also indicated. The putative hydrophobic signal peptide as well as the hydrophobic transmembrane domain are underlined. Panel C is a sequence alignment of Ag 2160 homologs that include mEGP, hEGP-2, hEGP-1. The hydrophobic signal peptide and the hydrophobic transmembrane domain are underlined. The protein sequences are aligned with the type I thyroglobulin sequence repeat (framed). Conserved cysteine residues are in bold type while highly conserved regions are indicated. Panel D is a Northern blot showing the tissue distribution of the Ag 2160 mRNA.
Panel A of Figure 7 shows inhibition of BUD cell growth by MAb 2160. BUD cells were plated and cultured for 5 days with the addition of 0-100 µg/ml of MAb 2160 (black, circles) or a non-relevant Ab (white, triangles) (A). On day 5, cell number and volume were determined. Panel B shows inhibition of BUD cell growth by the fusion protein P2160. Cells were cultured with or without 0-100 µg/ml of P2160 (black, circles) or a non-relevant fusion protein with an HIS-6 tag (white, triangles) and analyzed as in A. Each value represents mean ± SEM of 3 (A) or 2 (B) independent experiments, each run in triplicate ** P<0.01; *** P<0.001. Panel C depicts an immunoblot of immunoprecipitates prepared from the BUD cells treated with MAb 2160 (10 µg/ml) or P2160 (10 µg/ml). Two hours after the treatment, the BUD cells were lysed and immunoprecipitates were prepared using anti-Phospho-Ser/Thr/Tyr (IP P-Ser/Thr/Tyr) antibody or MAb 2160 (IP Ag 2160). Proteins from anti- P-Ser/Thr/Tyr immunoprecipitates were blotted with Mab 2160 (left panel), proteins from anti-Mab 2160 immunoprecipitates were blotted with anti- P-Ser/Thr/Tyr antibody.
Figure 8 depicts staining of frozen sections from rat embryos of day 9, 10, 12, 15 or 18 using MAb 2117.
Figure 9 depicts staining of frozen sections from adult rat MAb 2117.
Figure 10 depicts a partial cDNA clone of Ag 2117 that is recognized by Mab 2117.
Figure 11 is a Northern blot showing the tissue distribution of the Ag 2117 mRNA.

### MODE(S) FOR CARRYING OUT THE INVENTION

Throughout this disclosure, various publications, patents and published patent specifications are referenced by an identifying citation.

### Definitions:

The practice of the present invention will employ, unless otherwise indicated, conventional techniques of immunology, molecular biology, microbiology, cell biology and recombinant DNA, which are within the skill of the art. See, *e.g.*, Sambrook, et al. MOLECULAR CLONING: A LABORATORY MANUAL, 2^{nd} edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

As used in the specification and claims, the singular form "a", "an" and "the" include plural references unless the context clearly dictates otherwise. For example, the term "a cell" includes a plurality of cells, including mixtures thereof.

As used herein, the term "antibody" refers to a polypeptide or group of polypeptides which are comprised of at least one antibody combining site. An "antibody combining site" or "binding domain" is formed from the folding of variable domains of an antibody molecule(s) to form three-dimensional binding spaces with an internal surface shape and charge distribution complementary to the features of an epitope of an antigen, which allows an immunological reaction with the antigen. An antibody combining site may be formed from a heavy and/or a light chain domain (VH and VL, respectively), which form hypervariable loops which contribute to antigen binding. The term "antibody" includes, for example, vertebrate antibodies, hybrid antibodies, chimeric antibodies, altered antibodies, univalent antibodies, the Fab proteins, and single domain antibodies.

The term "monoclonal antibody" as used herein refers to an antibody composition having a substantially homogeneous antibody population. It is not intended to be limited as regards to the source of the antibody or the manner in which it is made. Monoclonal antibodies are highly specific, being directed against a single antigenic site. In contrast to conventional (polyclonal) antibody preparations which typically include different antibodies directed against different determinants (epitopes), each monoclonal antibody is directed against a single determinant on the antigen.

"A population of monoclonal antibodies" refers to a plurality of heterogeneous monoclonal antibodies, i.e., individual monoclonal antibodies comprising the population may recognize antigenic determinants distinct from each other.

An antibody "specifically binds" to an antigen if it binds with greater affinity or avidity than it binds to other reference antigens including polypeptides or other substances.

"Antigen" as used herein means a substance that is recognized and bound specifically by an antibody. Antigens can include peptides, proteins, glycoproteins, polysaccharides and lipids; portions thereof and combinations thereof.

The term "immunogen" is commonly known to artisans in the field. It is an antigen capable of stimulating production of an antibody when injected into a suitable host, usually a mammal. Compounds may be rendered immunogenic by many techniques known in the art, including crosslinking or conjugating with a carrier to increase valency, mixing with a mitogen to increase the immune response, and combining with an adjuvant to enhance presentation.

As used herein, the term "surface antigens" refers to the plasma membrane components of a cell. It encompasses integral and peripheral membrane proteins, glycoproteins, polysaccharides and lipids that constitute the plasma membrane. An "integral membrane protein" is a transmembrane protein that extends across the lipid bilayer of the plasma membrane of a cell. A typical integral membrane protein consists of at least one "membrane spanning segment" that generally comprises hydrophobic amino acid residues. Peripheral membrane proteins do not extend into the hydrophobic interior of the lipid bilayer and they are bound to the membrane surface by noncovalent interaction with other membrane proteins.

"Immunological reactivity" as applied to a cell or polypeptide refers to the ability of the cell or polypeptide to specifically bind to an antibody of the present invention. "Immunological reactivity" as applied to a population of monoclonal antibodies refers to the ability of the population to specifically bind to antigens representative of a particular cell type.

The term "heterologous" as applied to a cell used for immunization means that the cell is derived from a genotypically distinct entity from the recipient. For example, a heterologous cell may be derived from a different species or a different individual from the same species as the recipient. An embryonic cell derived from an individual of one species is heterologous to an adult of the same species.

A cell is of "ectodermal", "endodermal" or "mesodomal" origin, if the cell is derived, respectively, from one of the three germ layers - the ectoderm, the endoderm, or the mesoderm of an embryo. The ectoderm is the outer layer that produces the cells of the epidermis, and the nervous system. The endoderm is the inner layer that produces the lining of the digestive tube and its associated organs, including but not limited to pancreas and liver. The middle layer, mesoderm, gives rise to several organs (including but not limited to heart, kidney, gonads), connective tissues (e.g., bone, muscles, tendons), and the blood cells.

The terms "medium", "cell culture medium" and "culture medium" are used interchangeably. The terms refer to the aqueous environment in which the vertebrate cells are grown in culture. The medium comprises the physicochemical, nutritional, and hormonal environment. The cell culture medium is "serum-free", when the medium is essentially free of serum from any mammalian source, (e.g. sera from fetal bovine, horse, human, rabbit). By "essentially free" is meant that the cell culture medium comprises between about 0-5% serum, preferably between about 0-1% serum and most preferably between about 0-0.1% serum.

A "defined medium" refers to a medium comprising nutritional and hormonal requirements necessary for the survival and/or growth of the cells in culture such that the components of the medium are known. Traditionally, the defined medium has been formulated by the addition of nutritional and growth factors necessary for growth and/or survival. Typically, the defined medium provides at least one component from one or more of the following categories: a) all essential amino acids, and usually the basic set of twenty amino acids plus cystine; b) an energy source, usually in the form of a carbohydrate such as glucose; c) vitamins and/or other organic compounds required at low concentrations; d) free fatty acids; and e) trace elements, where trace elements are defined as inorganic compounds or naturally occurring elements that are typically required at very low concentrations, usually in the micromolar range. The defined medium may also optionally be supplemented with one or more components from any of the following categories: a) one or more mitogenic agents; b) salts and buffers as, for example, calcium, magnesium, and phosphate; c) nucleosides and bases such as, for example, adenosine and thymidine, hypoxanthine; and d) protein and tissue hydrolysates.

A "mitogenic agent" or "growth factor" is a molecule which stimulates mitosis of the mammalian cells. Generally, the mitogenic agent or growth factor enhances survival and proliferation of mammalian cells in cell culture and is a polypeptide. The mitogenic polypeptide can be a "native" or "native sequence" polypeptide (i.e. having the amino acid sequence of a naturally occurring growth factor) regardless of the method by which it is produced (e.g. it can be isolated from an endogenous source of the molecule or produced by synthetic techniques including recombinant techniques), or a variant or mutant thereof (see definition below). Preferably, the mitogenic polypeptide has the same amino acid sequence as a growth factor derived from a human, or a fragment thereof. Non-limiting examples include activators of one or more members of the erbB receptor family; agents which elevate cAMP levels in the culture medium (e.g. forskolin, cholera toxin, cAMP or analogues thereof); adhesion molecules such as neural cell adhesion molecule (N-CAM), laminin or fibronectin; progesterone; neurotrophic factors such as bone-derived neurotrophic factor (BDNF) and ciliary neuronotrophic factor (CNTF); neurotrophin-3, -4, -5, or -6(NT-3, NT-4, NT-5, or NT-6); or a nerve growth factor such as NGF- beta ; platelet-derived growth factor (PDGF); fibroblast growth factor such as acidic FGF (aFGF) and basic FGF (bFGF); vascular endothelial growth factor (VEGF); transforming growth factor (TGF) such as TGF- alpha and TGF- beta, including TGF- beta 1, TGF- beta 2, TGF- beta 3, TGF- beta 4, or TGF- beta 5; insulin-like growth factors, including IGF-I, IGF-II and des(1-3)-IGF-I (brain IGF-I); insulin-like growth factor binding proteins; and hormones such as estrogen, testosterone, thyroid hormone, insulin and any of those mitogens listed in Table 8.2 at pages 138-139 of Mather, J.P. and Roberts, P.E. (1998) "Introduction to Cell and Tissue Culture", Plenum Press, New York.

A "subject," or "individual" is used interchangeably herein, which refers to a vertebrate, preferably a mammal, more preferably a human. Mammals include, but are not limited to, rabbits, murines, simians, humans, farm animals, sport animals, and pets.

### Preparation of Immunogen

### Establishing serum-free cell cultures:

The immunogens of the present invention comprise a substantially homogenous population of viable and intact mammalian cells whose cell surfaces are free of serum and the cells are not human embryonic cells. Any mammalian cells capable of growth in culture are candidate immunogens. Cells suitable for *in vitro* culture may be derived from non human embryonic or adult tissues, normal or neoplastic tissues, tissues having a developmental origin of ectoderm, endoderm or mesoderm. Non-limiting examples of specific cell types that can now be grown in culture include connective tissue elements such as fibroblast, skeletal tissue (bone and cartilage), skeletal, cardiac and smooth muscle, epithelial tissues (e.g. liver, lung, breast, skin, bladder and kidney), neural cells (glia and neurones), endocrine cells (adrenal, pituitary, pancreatic islet cells), melanocytes, and many different types of haemopoietic cells. Cells in culture can be freshly isolated from body tissues (known as primary culture) or subcultured by expansion and/or cloning of the cells present in the primary culture (known as cell lines).

To ensure that the cell surfaces are free of serum, cells are typically grown in a defined medium that lacks serum but is supplemented with hormones, growth factors or any other factors necessary for the survival and/or growth of a particular cell type. Whereas a defined medium supporting cell survival maintains the viability, morphology, capacity to metabolize and potentially, capacity of the cell to differentiate, a defined medium promoting cell growth provides all chemicals necessary for cell proliferation or multiplication. The general parameters governing mammalian cell survival and growth *in vitro* are well established in the art. Physicochemical parameters which may be controlled in different cell culture systems are, e.g., pH, pO₂, temperature, and osmolarity. The nutritional requirements of cells are usually provided in standard media formulations developed to provide an optimal environment. Nutrients can be divided into several categories: amino acids and their derivatives, carbohydrates, sugars, fatty acids, complex lipids, nucleic acid derivatives and vitamins. Apart from nutrients for maintaining cell metabolism, most cells also require one or more hormones from at least one of the following groups: steroids, prostaglandins, growth factors, pituitary hormones, and peptide hormones to proliferate in serum-free media (Sato, G.H., et al. in "Growth of Cells in Hormonally Defined Media", Cold Spring Harbor Press, N.Y., 1982). In addition to hormones, cells may require transport proteins such as transferrin (plasma iron transport protein), ceruloplasmin (a copper transport protein), and high-density lipoprotein (a lipid carrier) for survival and growth *in vitro.* The set of optimal hormones or transport proteins will vary for each cell type. Most of these hormones or transport proteins have been added exogenously or, in a rare case, a mutant cell line has been found which does not require a particular factor.

The formulation of a defined medium for a specific cell type generally proceeds by three approaches that are widely known in the art. The first involves supplementation of existing basal nutritional media by adding various combinations of biomolecules performing serum functions: cell specific and non-cell specific hormones such as mitogens, transfer proteins, attachment and spreading factors (Barnes, D. and Sato, G. (1980) *Anal. Biochem.,* **102:**255). A variety of basal nutritional media are commercially available. Non-limiting examples of these minimal culture media include F12/DME, Ham's F10 (Sigma), Minimal Essential Medium (MEM, Sigma), RPMI-1640 (Sigma), Dulbecco's Modified Eagle's Medium (DMEM, Sigma) and Iscove's Modified Eagle's Medium (IMDM). In addition, any of the basal nutritional media described in Ham and Wallace (1979) *Meth. Enz.,* **58:**44, Barnes and Sato (1980) *Anal. Biochem.,* **102:**255, or Mather, J.P. and Roberts, P.E. (1998) "Introduction to Cell and Tissue Culture", Plenum Press, New York, can be used.

The testing of supplementing biomolecules, such as polypeptide factors in particular, is best done in a stepwise fashion testing new polypeptide factors in the presence of those found to be growth stimulatory. This is essential in some cases as polypeptide factor effects are seldom simply additive. Alternatively, some polypeptide factors can maintain cell survival or stimulate growth singly but the effects when added together cancel or are inhibitory. In general, cells require insulin and transferrin in a serum-free medium for optimal growth. These two factors should be tested first. Most cell lines also require one or more of the growth factors. These include but are not limited to epidermal growth factor (EGF), fibroblast growth factors (FGFs), insulin like growth factors I and II (IGFI, IGFII), nerve growth factor (NGF), heregulin, neuregulin, transforming growth factors (TGFs), platelet-derived growth factors (PDGFs), interleukins (ILs) and other hemopoietic cell growth factors.

The second approach for establishing a defined medium suitable for culturing a particular cell type proceeds with new formulations of existing nutritional media by lowering the serum concentration, until cell growth is limited, and then adjusting the concentration of each component of the nutrient medium until growth resumes (Ham, R.G. and McKeehan, W.L. (1979) *Academic Press,* **53**:44; Ham, R.G. (1981) *Handbook of Experimental Pharmacology,* **57**:13). Employing these two techniques, numerous mammalian cell lines derived from embryonic or adult organs, normal or neoplastic tissues, cells of ectodermal, mesodermal or endodermal origin, have been established in serum-free medium. Freshney, R. I. (1986) "Animal Cell Culture, A Practical Approach", IRL press summarizes the medium conditions for 44 serum-free cultures of non-transformed cells, including fibroblasts, epithelial cells, neuronal cells, hematopoietic cells, lymphoid cells, as well as transformed cells derived from a diversity of carcinoma, adenocarcinoma, and neuroblastoma. Sato, G. H., et al. (1982) "Growth of Cells in Hormonally Defined Media" details procedures for culturing cells of endocrine, exocrine, mammary, neural, or reproductive-tract origin in serum-free media.

Because media formulated for one cell type will generally support growth of other lines and primary cultures of independent origin if they are of the same cell type (Li, R. H. et al. (1996) *J. Neurosci. Methods,* **67:**57-69; Li, R. H. et al. (1996) *J. Neuroscience* **16(6):**2012-2019; Levi, A.D.O. et al. (1997) *Experimental Neurology* **143**:25-36), another well-established approach involves testing a defined medium that is most closely related to one already found to support survival and/or growth of a similar type of cells. The vast wealth of information on nutritional and hormonal requirements for a diversity of cell types has greatly facilitated artisans to routinely search for a set of medium conditions necessary for growth or survival of any given cell types. In establishing a serum-free cell culture, any one of the aforementioned approaches, or procedures modified therefrom can be employed either singly or in any combination.

Accordingly, the present invention provides various cell lines established in serum-free media that are subsequently used as immunogens for generating a population of monoclonal antibodies recognizing antigens representative of these cell lines. In one embodiment, the invention provides embryonic Schwann cells (ESC) and adult Schwann cells (ASC) derived from rat dorsal root ganglia (U.S. Patent No. 5,721,139; U.S. Patent No. 5,714,385; Li, R. (1997) *Endocrinology,* **138**:2648-2657). In another embodiment, the invention includes two pancreatic epithelial cell lines established from primary cultures of dissected rat e12 embryonic pancreatic buds (BUD) and rat e 17 ductal epithelium (RED). In yet another embodiment, the invention provides bronchiolar epithelial cells of rat lung such as the RL-65 cell line. In yet another embodiment, the invention provides undifferentiated granulosa cell line derived from rat ovarian follicles (ROG). In still yet another embodiment, the invention provides neonatal lung epithelial cell line BR516, rat early embryonic (day 9) neuroepithelial cell line NEP, and the cell line NODD derived from non-obese diabetic mouse pancreatic ductal epithelial cells. Methods for generating the RL-65, ROG, BR516, NEP cells are described in U.S. Patent No. 5,364,785; Li, R., et al. (1997) *Endocrinology,* 138(7):2648-2657; Roberts, P. E. (1992) *Animal Cell Techology: Basic and Applied Aspects,* 335-341; Roberts, P. E. (1990) *Am. J. Physiol.,* **3**:L415-L425); and Li, R. H. et al. (1996) Endocrine **5:**205-217. Procedures for establishing the NODD cells in a serum-free medium are essentially the same as that applied to the RED cells described herein (see Example 1).

### Maintaining cell viability:

The immunogens of the present invention comprise viable and intact cells. Viable cells can be grown as a monolayer anchored onto a solid phase substrate, or as aggregates in a suspension culture. The choice of substrate is determined largely by the type of cells. Most cells can be propagated on a substrate made of e.g., glass, plastic or ceramic material. For certain cell types, such as neurons, epithelial and muscle cells, substrates precoated with charged substances that enhance cell attachment and spreading are preferred. Commonly employed coating materials include biological substrates that bear a net positive charge. Non-limiting examples of biological substrates include extracellular matrix/adhesion proteins such as laminin, fibronectin, collagen, or synthetic polypeptide such as poly-lysine. A variety of non-biological substrates such as membranes made of nitrocellulose, nylon, polytetrafluoroethylene, or any other implant materials can also be used to support growth of cells in a serum-free medium.

Precautions are taken to maintain membrane integrity and preserve cell membrane components when harvesting cells cultured on different substrates. Unlike the traditional method of dissociating the anchored cells or cell layers by the action of strong proteolytic enzymes such as serine proteinase, trypsin, cell immunogens of the present invention are typically removed from the culture substrates by agents that minimize damages to the cell surface antigens. These agents include chelating agents, such as EDTA and EGTA, which bind to divalent metal ions (e.g. calcium and magnesium) known to be necessary for cell-substrate attachment. Other suitable cell dissociation agents encompass collagenases, dispases, and neutral proteinases when used in conjunction with serine proteinase inhibitors (e.g. soybean trypsin inhibitor). Treatment of cells with these agents mostly result in disruption of the extracellular matrix components while preserving the cell surface proteins. The time required to detach the cells anchored on a solid substrate can vary depending on the protease enzymes chosen, but will normally be a period of about 3 minutes to 30 minutes, and preferably about 5 minutes to 15 minutes. The enzymatic treatment can be carried out at room temperature or at about 37 °C. Excess enzyme can be removed by gentle washing with buffers having pH and salt concentrations in the physiological range that are routinely prepared by one skilled in the art.

Prior to immunization, cell viability may be confirmed by the measurement of membrane integrity. The methods for assessing membrane integrity are known in the art. The most common assay involves staining cells with a dye that reacts with either living or dead cells. As is apparent to one skilled in the art, exemplary dyes include trypan blue, eosin Y, naphthalene black, nigrosin, erythrosin B and fast green.

Where desired, cell immunogens may be mixed with adjuvants to enhance host immune response. Suitable adjuvants are aluminum hydroxide, alum, QS-21 (U.S. Pat. No. 5,057,540), DHEA (U.S. Pat. Nos. 5,407,684 and 5,077,284) including its precursors and modified forms, (e.g., DHEA-S, the sulfonated form of DHEA), β-2 microglobulin (WO 91/16924), muramyl dipeptides, muramyl tripeptides (U.S. Pat. No. 5,171,568) and monophosphoryl lipid A (U.S. Pat. No. 4,436,728; WO 92/16231) and its derivatives, e.g., Detox^{™}, and BCG (U.S. Pat. No. 4,726,947). Other suitable adjuvants include, but are not limited to, Freund's adjuvant, aluminum salts, squalene mixtures (SAF-1), muramyl peptide (MDP), saponin derivatives, mycobacterium wall preparations, mycolic acid derivatives, nonionic block copolymer surfactants, Quil A, cholera toxin B subunit, polyphosphazene and derivatives, and immunostimulating complexes (ISCOMs) such as those described by Takahashi et al. (1990) *Nature* 344:873-875. A preferred adjuvant is Ribi Adjuvant System manufactured by Ribi Immunochem Research, Inc.

### Immunization and generation of hybridomas:

The route and schedule of immunization of the host animal are generally in keeping with established and conventional techniques for antibody stimulation and production.

While mouse was employed as the test mode, it is contemplated that any mammalian subject including humans or antibody producing cells therefrom can be manipulated according to the processes of this invention to serve as the basis for production of mammalian, including human, hybridoma cell lines. Typically, the host animal is inoculated intraperitonealy with an immunogenic amount of the cells and then boosted with similar amounts of the immunogen. In an alternative, cells grown on non-biological membrane matrix, are surgically implanted intraperitonealy into the host animal. Lymphoid cells, preferably spleen lymphoid cells from the host, are collected a few days after the final boost and a cell suspension is prepared therefrom for use in the fusion.

Hybridomas are prepared from the lymphocytes and immortalized myeloma cells using the general somatic cell hybridization technique of Kohler, B. and Milstein, C. (1975) *Nature* **256**:495-497 as modified by Buck, D. W., et al., (1982) *In Vitro,* **18**:377-381. Available myeloma lines, including but not limited to X63-Ag8.653 and those from the Salk Institute, Cell Distribution Center, San Diego, Calif., USA, may be used in the hybridization. Basically, the technique involves fusing the myeloma cells and lymphoid cells using a fusogen such as polyethylene glycol, or by electrical means well known to those skilled in the art. After the fusion, the cells are separated from the fusion medium and grown in a selective growth medium, such as HAT medium, to eliminate unhybridized parent cells. Any of the media described herein, supplemented with or without serum, can be used for culturing hybridomas that secrete monoclonal antibodies. As another alternative to the cell fusion technique, EBV immortalized B cells are used to produce the monoclonal antibodies of the subject invention. The hybridomas are expanded and subcloned, if desired, and supernatants are assayed for anti-immunogen activity by conventional immunoassay procedures (e.g., radioimmunoassay, enzyme immunoassay, or fluoroescence immunoassay).

Hybridomas encompass all derivatives, progeny cells of the parent hybridomas that produce monoclonal antibodies specific for antigens representative of the type of cells used for immunization.

Hybridomas that produce such antibodies may be grown *in vitro* or *in vivo* using known procedures. The monoclonal antibodies may be isolated from the culture media or body fluids, by conventional immunoglobulin purification procedures such as ammonium sulfate precipitation, gel electrophoresis, dialysis, chromatography, and ultrafiltration, if desired. Undesired activity if present, can be removed, for example, by running the preparation over adsorbants made of the immunogen attached to a solid phase and eluting or releasing the desired antibodies off the immunogen.

### Characterization and selection of monoclonal antibody:

Immunization of a host animal with a plurality of intact and viable cells that are free of serum yields a population of monoclonal antibodies exhibiting the following characteristics: (a) lacks substantial immunological reactivity with serum biomolecules; (b) binds to surface antigens that are representative of the type of cells used for immunization; and (c) contains at least one monoclonal antibody reactive with an antigen, which is selectively expressed in certain body tissues (tissue-selective), localized to a specific region (sub-tissue selective) or a particular cell layer within those tissues (cell-type specific).

The lack of substantial immunological reactivity is determined by testing the population of monoclonal antibodies against total serum biomolecules. A population of monoclonal antibodies is deemed to lack substantial immunological reactivity if it yields no detectable binding to total serum biomolecules when used with a dilution about 1:10,000, preferably about 1:1000, more preferably about 1:500. The total serum may be tested at a concentration about 0.001% (v/v), preferably at a concentration about 0.01 %, more preferably at 0.1% and even more preferably at 1%. Antigen binding can be detected by immunoassays including, e.g, ELISA and immunoblotting assays. Preferably, the detection is carried out by immunoblotting total serum biomolecules resolved by electrophoresis on a reducing polyacrylamide gel.

The ability of the population of monoclonal antibodies to recognize surface antigens representative of a specific cell type, can be tested against viable and intact cells of that particular type, which present surface antigens exhibiting their native configurations. For example, antibodies bound to the surface antigens can be detected directly by immunoassays, for example, by reacting labeled antibodies with viable and intact cells immobilized onto a substrate. In an alternative, binding to surface antigens can be assessed by cell sorting, which involves labeling target cells with antibodies coupled to a detectable agent, and then separating the labeled cells from the unlabeled ones in a cell sorter. A sophisticated cell separation method is fluorescence-activated cell sorting (FACS). Cells traveling in single file in a fine stream are passed through a laser beam, and the fluorescence of each cell bound by the fluorescently labeled antibodies is then measured.

Immunoassays and cell sorting techniques such as FACS can also be employed to isolate monoclonal antibodies that are tissue-selective, sub-tissue selective or cell-type specific. A tissue-selective monoclonal antibody binds to an antigen that is not ubiquitously expressed in all body tissues from a subject. The types of body tissues include but are not limited to pancreas, esophagus, lung, kidney, colon, stomach, brain, liver, heart, ovary, skin, breast, muscle, bone, uterus, bladder, spinal cord, and various kinds of body fluids. A monoclonal antibody is sub-tissue selective if it binds to a target antigen localized to certain regions within a tissue. Most body tissues are complex structures assembled by layers of cells of various types. A cell-type specific monoclonal antibody reacts with an antigen that is exclusively expressed in certain cell layers or cell types within a single tissue or the developmentally related tissues thereof. Exemplary cell-type specific monoclonal antibodies are those that specifically bind to one of the following cell types: epithelial cells, endothelial cells, neurons, Schwann cells, muscle cells, erythrocytes, lymphocytes, germ cells, glial cells, astrocytic cells, and mesenchymal cells. The tissue selectivity of a monoclonal antibody is generally examined by immunohistochemical analysis, in which frozen or fixed tissue sections and/or tissue homogenates are stained with such antibody at various concentrations. The sub-tissue selectively of a monoclonal antibody can be assessed by comparing the staining patterns of various sections of the tested tissue. A cell-type specific monoclonal antibody are conveniently identified by immunblotting the crude lysates of cells of distinct types, or sorting various types of cells based on a specific binding of the tested monoclonal antibody to the surface antigens that are native to a specific cell type. Cell sorting technique such as FACS is particularly applicable for isolating monoclonal antibodies that bind to the extracellular domain of a surface antigen. Procedures for conducting immunoassays and cell-sorting are well established in the art and thus they are not detailed herein.

The monoclonal antibodies can be bound to many different carriers. Carriers can be active and/or inert. Examples of well-known carriers include polypropylene, polystyrene, polyethylene, dextran, nylon, amylases, glass, natural and modified celluloses, polyacrylamides, agaroses and magnetite. The nature of the carrier can be either soluble or insoluble for purposes of the invention. Those skilled in the art will know of other suitable carriers for binding antibodies, or will be able to ascertain such, using routine experimentation.

The monoclonal antibodies can also be conjugated to a detectable agent or a hapten. The complex is useful to detect the antigens to which the antibody specifically binds in a sample, using standard immunochemical techniques such as immunohistochemistry as described by Harlow and Lane (1988) *supra.* There are many different labels and methods of labeling known to those of ordinary skill in the art. Examples of the types of labels which can be used in the present invention include radioisotopes, enzymes, colloidal metals, fluorescent compounds, bioluminescent compounds, and chemiluminescent compounds. Those of ordinary skill in the art will know of other suitable labels for binding to the antibody, or will be able to ascertain such, using routine experimentation. Furthermore, the binding of these labels to the antibody of the invention can be done using standard techniques common to those of ordinary skill in the art.

Another technique which may also result in greater sensitivity consists of coupling the antibodies to low molecular weight haptens. These haptens can then be specifically detected by means of a second reaction. For example, it is common to use such haptens as biotin, which reacts avidin, or dinitropherryl, pyridoxal, and fluorescein, which can react with specific anti-hapten antibodies. See Harlow and Lane (1988) *supra.*

The monoclonal antibody populations and hybridomas producing such monoclonal antibodies of the present invention can have diagnostic and/or therapeutic applications.

Applying the above-described general techniques, a population of monoclonal antibodies reactive with antigens representative of embryonic pancreatic ductal cells was generated. Of a pool of 15 different monoclonal antibodies examined, thirteen of them recognize distinct antigens on the cell surfaces as determined by cross-competition assays for binding to intact, non-permeabilized cells. FACS analysis further confirmed that the antibodies are directed to the extracellular domain of the cell surface antigens. Immunohistochemical analyses conducted with two exemplary monoclonal antibodies, Mab 2160 and Mab 2117, have revealed their selectivity for staining certain body tissues, sub-tissue structures, and particular layers of cells within a tissue.

### Isolation and Identification of the target antigen:

The monoclonal antibodies provide specific reagents for isolating and cloning the target antigens. As used herein, the term "isolated" means separated from constituents, cellular and otherwise, in which the antigens or fragments thereof, are normally associated with in nature.

The surface antigen recognized by a monoclonal antibody of the present invention can be isolated by a number of processes well known to artisans in the field. Representative procedures are immunoprecipitation and immunoaffinity purification of the target antigens from tissue homogenates or cell lysates. Both methods proceed with binding the target antigens to the monoclonal antibodies that are immobilized onto a solid-phase matrix (e.g. protein A and protein G sepharose beads), followed by separating the bound antigens with the unbound proteins, and finally eluting the antigens from the antibody-coupled solid-phase matrix. Subsequent analysis of the eluted antigens may involve electrophoresis for determining the molecular weight, and protein sequencing for delineating the amino acid sequences of the target antigen. Based on the deduced amino acid sequences, the cDNA encoding the antigen can then be obtained by recombinant cloning methods including PCR, library screening, homology searches in existing nucleic acid databases, or any combination thereof. Commonly employed databases include but are not limited to GenBank, EMBL, DDBJ, PDB, SWISS-PROT, EST, STS, GSS, and HTGS.

A preferred method of cloning the target surface antigen is by "panning" the antibodies for cells expressing the cell surface antigen of interest. The "panning" procedure is conducted by obtaining the cDNAs of cells that express the antigen of interest, over-expressing the cDNAs in a second cell type, and screening cells of the second cell type for a specific binding to the monoclonal antibody.

cDNAs can be obtained by reverse transcribing the mRNAs from a particular cell type according to standard methods in the art. Specifically, mRNA can be isolated using various lytic enzymes or chemical solutions according to the procedures set forth in Sambrook et al. ("Molecular Cloning: A Laboratory Manual", Second Edition, 1989), or extracted by nucleic-acid-binding resins following the accompanying instructions provided by manufacturers. The synthesized cDNAs are then introduced into an expression vector to produce the antigens in cells of a second type. It is implied that an expression vector must be replicable in the host cells either as episomes or as an integral part of the chromosomal DNA. Suitable expression vectors include plasmids, viral vectors, including adenoviruses, adeno-associated viruses, retroviruses, cosmids, etc.

The vectors containing the polynucleotides of interest can be introduced into the host cell by any of a number of appropriate means, including electroporation, transfection employing calcium chloride, rubidium chloride, calcium phosphate, DEAE-dextran, or other substances; microprojectile bombardment; lipofection; and infection (where the vector is an infectious agent, such as vaccinia virus, which is discussed below). The choice of introducing vectors or polynucleotides will often depend on features of the host cell.

Any host cells capable of over-expressing heterologous DNAs can be used for the purpose of isolating the genes encoding the target surface antigens. Non-limiting examples of mammalian host cells include but not limited to COS, HeLa, and CHO cells. Preferably, the host cells express the cDNAs at a level of about 5 fold higher, more preferably 10 fold higher, even more preferably 20 folds higher than that of the corresponding endogenous antigens, if present, in the host cells. Screening the host cells for a specific binding to the selected monoclonal antibodies is effected by an immunoassay or preferably, FACS. By identifying the individual target antigens, the combination of cell surface antigens expressed in a specific cell type can then be determined.

The following examples provide a detailed description of the preparation, characterization, and use of representative monoclonal antibodies of the present invention. These examples are not intended to limit the invention in any manner.

### EXAMPLES

### MATERIALS AND METHODS:

### Example 1 Culture of embryonic pancreatic ductal epithelial cells in serum-free medium

Embryonic ducts were isolated using a modification of a previously described procedure (Githens, S., et al. (1989) **25**:679-688). To generate the RED cell line, 2-, 3-, or 18-day pregnant Sprague Dawley rats were sacrificed by CO₂ asphyxiation. The embryos were transferred to ice cold Hank's Balanced Salt Solution, containing 20 µg/ml gentamycin. The embryonic pancreati were removed on ice under a dissecting microscope and placed in F12/DME. To each 12-15 pancreati in 1ml of F12/DME, 25 µl of a Collagenase-dispase solution (50 mg/ml) containing soybean trypsin inhibitor (1 mg/ml) was added. The dish was then incubated at 37°C for 30 min with frequent pipetting to break up tissue into smaller fragments. The digest was washed by centrifugation after layering on top of a 5% BSA gradient. Further dissociation was accomplished by filtration through a tissue sieve or through 200 mesh Nitex cloth.

Tissue fragments, mostly ducts, were washed by centrifugation at 800x g for 6 min in F12/DME and then resuspended in growth medium which consisted of F12/DME supplemented with 14F: rhu-insulin (10 µg/ml), transferrin (10 µg/ml), EGF (10 ng/ml), ethanolamine (1 µM), aprotinin (25 µg/ml), glucose (5 mg/ml), phosphoethanolamine (1 µM), triiodothyronine (5 pM), selenium (25 nM), hydrocortisone (0.5 µM), progesterone (10 nM), forskolin (1 µM), heregulin β177-244 (10 nM) and bovine pituitary extract (BPE) (5 µl/ml, 75 µg/ml protein). The cell suspension was then distributed evenly to either fibronectin-coated or collagen-coated 24-well plates. Cyst-like structures formed within 48-72 hr in culture. These were removed with the supernatant washed, resuspended in the 14F growth medium and replated onto either collagen or fibronectin-coated plates. The cyst-like structures attached and begin to spread within 24 hr. After 5-7 days, these cultures were 75% confluent, whereupon they were subcultured at a 1:2 split ratio by dissociation in trypsin-EDTA, neutralized with 1 mg/ml soybean trypsin inhibitor, washed by centrifugation, resuspended in 14F growth medium and plated on fibronectin-coated plates. Thereafter, the cultures were split every 3-4 days at a high split ratio (1:3 to 1:5). Fibroblast contamination was minimal and eliminated altogether by serial cloning in 15% self conditioned medium in 96-well microtiter plates (Mather, J. P. and Sato, G. H. (1979) *Exp. Cell. Physiol.,* **124**:215-221; Roberts, P. E., et al. (1990) *Am. J. Physiol.,* **3**:L415-L425).

BUD cultures were established from 12 day pregnant Sprague Dawley rats. After dissecting out the embryos, the dorsal and ventral pancreatic evaginations were surgically dissected and cultured in separate wells of a 48 well dish without initial enzymatic dissociation of the tissue. The cells were carried as described above. The BUD and RED cells have both been in continuous culture for at least 80 population doublings. They have maintained a normal karyotype, are confirmed to be of rat origin and free of mycoplama.

### Example 2 Generation of monoclonal antibodies against BUD/RED cell surface proteins

Balb/c mice were immunized with 5 X 10⁶ intact BUD or RED cells, without adjuvant, weekly for 10 to 15 weeks. Alternatively, cells grown on nitrocellulose discs were surgically implanted intraperitonealy every 2 weeks for 6 weeks. Sera from the immunized mice were tested for antibodies to BUD and RED cells, by FACS analysis of binding, as described below. The mice with the highest titers were given an additional boost of 5 X 10⁶ cells. Three days later, the lymphocytes from the mouse spleen were fused with the mouse myeloma line X63-Ag8.653 using 50% polyethylene glycol 4000 according to the procedure described elsewhere (Oi, V. and Herzenberg, L. (1980) "Immunoglobulin-Producing Hybrid Cell Lines"). Fused cells were plated at a density of 200,000 cells per well in 96-well tissue culture plates and hybridomas were selected using HAT media supplement (Sigma, St. Louis, MO). On day 10 following the fusion, the hybridoma supernatants were screened for the presence of BUD/RED specific Mabs by FACS. The hybrids producing MAbs that bound to BUD and RED cell lines were then screened against the TR-1 rat endothelial cell line. Selected hybridomas were cloned by limiting dilution to produce stable hybridomas. MAbs were produced in ascites and the antibodies were purified on protein A-Sepharose columns (Fermentech, Inc., Edinburgh, Scotland) and stored sterile in PBS at 4°C.

### Example 3 FACS Analysis

Cells were detached from tissue culture flasks in the presence of 0.5 mM EDTA for 15 min, treated for 10 min with collagenase/dispase (Boehringer Mannheim, indianapolis, IN), centrifuged at 1400 rpm for 5 min and resuspended in PBS containing 1% BSA and 2 mM EDTA (FACS diluent). The cells were counted, adjusted to 10⁷ cells/ml and 0.1 ml of cells were incubated with 1 µg of purified MAbs in 100 µl FACS diluent for 30 min at 4°C. The samples were washed, resuspended in 0.1 ml diluent and incubated with 1µg of FITC conjugated F(ab')₂ fragment of goat anti-mouse IgG for 30 min at 4°C. The cells were washed, resuspended in 0.5 ml FACS diluent and analyzed using a FACScan cell sorter (Becton Dickinson, Mt. View, CA).

The antibodies were screened by FACS for binding to various other cell lines in addition to the BUD and RED lines. These include the following: RIN-M and RIN-F rat insulinoma cell lines (Gazdar, A. F., et al. (1980) *Proc. Natl. Acad Sci.,* **77**:3519-3523); ARIP rat acinar tumor cell line (Jessop, N. W. and Hay, R. J. (1980) *In Vitro,* **16**:212); NODD mouse adult pancreatic ductal cell line (established in this lab by the same method used for RED cells but starting from adult NOD mouse pancreas); BR516 lung epithelial cell line (Roberts, P. E. et al. (1992) *Animal Cell technology: Basic and Applied Aspects,* 335-341; Roberts, et al. (1990) *Am. J. Physiol.,* **3**: L415-L425); rat adult (ASC) and embryonic (ESC) Schwann cell lines (Li, R. H. et al. (1996) *J. Neurosci. Methods,* **67:**57-69), RAT-1 rat fibroblast cell line (Botchan, M., et al. (1976) *Cell,* **9:**269-287); TR-1 rat capillary endothelial cell line (Mather, J. P., et al. (1982) *Annals of the New York Academy of Sciences,* **383:**44-68); TR-M rat peritubular myoid cell line (Mather, J. P., et al. (1982) *Annals of the New York Academy of Sciences,* **383:**44-68), and primary neonatal rat cardiomyocyte cultures rCM (Lai, J., et al. (1996) *Am. J. Physiol.,* **271**:H2197-H2208). All cell lines were carried in F12/DMEM medium supplemented with 10% fetal calf serum (ARIP, RIN-F, RIN-M, RAT-1, TR-1, TR-M) or the published hormone supplements appropriate to the cell line (BR516, NODD, ASC, ESC, rCM).

### Example 4 Immunohistochemical analysis

Embryos were snap-frozen in liquid nitrogen immediately after removal from 9, 10, 12, 15, or 18-day pregnant Sprague Dawley rats and stored at -70°C until sectioning. Sections of 4-6 µm thickness were cut on a cryostat, air-dried, fixed in acetone for 5 min and air-dried overnight. BUD and TR-1 cell monolayers were fixed with 4% paraformadehyde. After quenching of the endogenous peroxidase using the glucose oxidase/glucose method (Andrew and Jasani, 1987), blocking of the endogenous biotin using an avidin/biotin blocking kit (Vector, Burlingame, USA) and blocking the endogenous immunoglobulin binding sites with PBS/1% BSA (25 min), either the sections or the cells were overlaid for 2 hr with purified MAbs 2160, 2161, 2115 or 2117 (4.8 µg/ml, 1.66 µg/ml, 2.1 µg/ml and 1.8 ug/ml, respectively, in PBS/1% BSA). Subsequently, samples were incubated with rhodamine-conjugated anti-mouse IgG (Chemicon, Temecula, CA) or biotin-rat anti-mouse IgG1 (1:500)(Zymed, San Francisco, CA) for 2 hr and peroxidase-conjugated streptavidin (4 mg/ml)(Jackson, West Grove, FL) for 30 min. After several rinses in PBS, immunostaining was developed for 10-15 min with 3-amino-9-ethylcarbazole (Dako, Carpinteria, CA). Sections were counterstained with Mayer's hematoxylin and mounted in glycergel (Dako). When conducting immunohistochemical analysis of e9, e10, e 12, e15, and e18 embryo, 2 separate experiments were performed and at least 4 sections examined from each embryo for each MAb or control.

Guts were dissected from 12.5-day rat embryos and fixed in 3% paraformaldehyde overnight. After washes, permeabilisation with acetone at -20 °C during 7 min and blocking the endogenous immunoglobulin binding sites with PBS/1% BSA/1% DMSO/2% goat serum, tissues were incubated overnight either with a rabbit polyclonal anti-rat PDX1 (1:1000) or the MAb 2160 (1:400). Immunostaining was analyzed after an overnight incubation either with the secondary antibody Cy3-conjugated affinity purified goat anti-mouse or rabbit IgG.

### Example 5 Isolation of mRNA and construction of expression library

Messenger RNA was isolated directly from cultured BUD cells using the In Vitrogen FastTrack 2.0 mRNA Isolation System. Oriented cDNA transcripts were prepared from 5 mg poly-(A)⁺ mRNA using the Gibco-BRL SuperScript Plasmid System and fractionated on 5% acrylamide-TBE slab gel. Eluted cDNAs were ligated into the Xho I-Not I sites of the mammalian expression vector pRK5D, and then electroporated into Gibco-BRL DH10B cells under conditions recommended by the manufacturer.

### Example 6 Recovery of cDNA clones encoding the target antigens by panning

Screening of the BUD cell library was carried out using a modified version of a technique previously described (Seed and Aruffo, 1987). Briefly, the cDNA library was transfected into COS cells by electroporation (Neumann, E., et al. (1982) *EMBO J.,* **7**:841-845). After 2 days of culture; transfected COS cells were resuspended, then incubated with a pool of the antibodies shown in table I at a concentration of 2 mg/ml each and replated onto dishes coated with affinity-purified rabbit anti-mouse IgG and IgM. A Hirt supernatant was prepared from adherent cells and used to transform competent escherichia coli. After amplification, bacterial colonies were harvested, then plasmid cDNA was isolated by alkaline miniprep method (Birnboim, H. C. and Doly, J. (1979) *Nucleic Acids Research,* **7**:1513-1523) and transfected into COS cells to performed a new round of immunoselection. After 3 rounds of panning with the pooled antibodies, subsequent rounds of panning were performed on the individual purified MAbs

### Example 7 Gene sequence analysis

ABI Dye-terminator TM chemistry (PE Applied Biosystems, Foster City, USA) was used to sequence the clone 2160 with a primer walking strategy (Sanger, F., et al. (1977) *Proc. Natl. Acad Sci.,* **74**:5463-5467). The sequences were collected with an ABI377 instrument (PE Applied Biosystems, Foster City, CA). The sequences generated by the different walking primers for both DNA strain were edited and assembled in the Sequencher TM (Gene Codes Corp, Ann Arbor, MI). All sequence analysis in database were performed in the in-house sequence analysis program (Genentech Inc., South San Francisco, CA). The program ALIGN (Dayhoff, M. O., et al. (1983) *Methods Enzymol.,* **91**:524-545) was used to analyze the relationship between the clone 2160, mEGP, hEGP-1 and hEGP-2.

### Example 8 Immunoblotting cell lysates

Untreated, MAb 2160 (10 µg/ml) or P2160 (10 vg/ml) treated BUD and RED cells were either lysed in PBS/1% NP40/0.5% deoxycholate/0.1% SDS/5mM EDTA and the lysate was loaded on a 4-20% Novex Tris-Glycine gel or lysed in a buffer containing 10 mM Tris pH 8.0, 150 mM Sodium chloride, 1% sodium deoxycholate, 1% (v/v) triton-X-100, 0.1% sodium dodecylsulfate, I mM leupeptin and 1 mM PMSF, and the lysate was immunoprecipitated with an anti-phospho-Ser/Thr/Tyr monoclonal antibody (Clontech) or MAb 2160, boiled and loaded on a 4-12% Novex Tris-Glycine gel. The gel was run at 100V and electroblotted for 60 min at 0.5 Amp onto a Protran nitrocellulose membrane (Schleicher and Schuell, Keene, NH). The membrane was blocked in PBS/5% non-fat milk/0.5% between 20/0.01 % Thimerosal (assay buffer) for 1 hr at room temperature. The blot was washed in PBS/0.05% Tween 20 (PBST), and incubated with each MAb (1 µg/ml), an anti-phospho-Ser/Thr/Tyr monoclonal antibody (Clontech) or antibodies against pancreatic markers (cytokeratin 7 (1:500), PDX1 (1:500), carboxypeptidase A (1:500), tyrosine hydroxylase (1:1000)) for 1 hr. The membrane was washed with PBST and incubated for an additional 1 hr with a 1:5000 dilution of goat anti-mouse IgG or anti-rabbit IgG peroxidase. The membrane was washed thoroughly and developed using the Amersham ECL chemiluminescence system (Amersham, Arlington Heights, VA).

### Example 9 Northern blot Analysis

Poly-(A)⁺ RNA blots from the indicated human and rat adult tissues were purchased from Clontech (Palo Alto, CA) and hybridized to a 1.29 Kb (γ³²P)dCTP cDNA probe for clone 2160, labeled by random priming (2×10⁶ cpm/ml) (Feinberg and Vogelstein, 1983). After a 1 hr hybridization, membranes were wash at 65°C in 0.1xSSC/0.1% SDS and subjected to autoradiography at 70°C.

### Example 10 Production of the fusion proteins 2160 (P2160) extracellular domains (ECD) HIS-6

Specific PCR primers were synthesized on the basis of the DNA sequence of protein 2160 extracellular domains. A HIS-6 tag sequence was added to each of the C-terminal primers for affinity purification purposes. The p2160 ECD cDNAs were generated by PCR and inserted into pRK5, an expression plasmid using the cytomegalovirus promoter/enhancer with simian virus 40 (SV40) termination and polyadenylation signals located downstream of the inserted cDNA. These constructs were transiently transfected into human embryonic kidney 293 cells using Lipofectamine. The expressed proteins were purified using a chelating Sepharose column charged with nickel (Amersham Pharmacia Biotech, Piscataway, N.J.). Protein concentration was determined by OD 280.

### RESULTS AND DISCUSSIONS:

### BUD and RED embryonic pancreatic ductal epithelial cell lines

Two pancreatic epithelial cell lines were established from primary cultures of dissected rat e 12 embryonic pancreatic buds (BUD) and rat e17 ductal epithelium (RED), respectively. The cultures were initiated and carried in a serum-free medium optimized to select for the growth of the epithelial cells. Each component of the 14F medium contributes to the optimal growth of the cells (Table I). Under these conditions, the fibroblast and mesenchymal cells are lost from the cultures within 2 passages and the remaining cells are uniformly epithelial. The cultures have a log phase population doubling time of 11.4 hr and 14 hr for BUD and RED cells, respectively. The cells form a contact-inhibited monolayer, have a normal karyotype and have been grown continuously for over 80 population doublings with no obvious change in cell morphology or growth profile. In accordance with previous work establishing rodent cell lines in this fashion (Loo, D., et al. (1989) *J. Cell. Physiol.,* **139:**484-491; Roberts, P. E., et al. (1990) *Am. J. Physiol.,* **3**:L415-L425), no cell senescence has been observed.

In order to better characterize the BUD and RED cell lines, the presence of various proteins known to be present at the early stage of pancreatic development was investigated by Western blot analysis (Figure 1). We demonstrate that the BUD cells and, to a lesser extend, the RED cells express cytokeratin 7 (MW 54 kD), which is present only in the pancreatic ductal epithelium (Bouwens, L. (1998) *J. Pathol.,* **184:**234-239). BUD and RED cells express carboxypeptidase A (35 kD), other ductal marker (Kim, S. K., et al. (1997) *Development,* **124**:4243-4252). The procarboxypeptidase (45 kD) is also present in the two pancreatic cell lines. Both pancreatic BUD and RED cell lines also express the homeodomain-containing transcription factor for insulin gene expression PDX1 (42kD), which appears before insulin during the ontogeny of the mouse pancreas (Watada, H., et al. (1996) *Diabetes,* **45:**1826-1831). The tyrosine hydroxylase (60 kD), an early islet progenitor (Teitelman, G. and Lee, J. K. (1987) *Dev. Biol.,* **121:**454-466) and ductal marker, was detected in the BUD cells and to a lesser extend in the RED cells.

### Monoclonal antibodies to BUD/RED cell surface proteins

Intact, viable BUD and RED cells were used to immunize mice and generate monoclonal antibodies (MAbs). Ten monoclonal antibodies were selected based on their binding to the RED and BUD cells (Table IIA). Mabs 2116, 2117, and 2140 were found to react with the TR-1 endothelial cell line (Mather, J. P., et al. (1982) *Annals of the New York Academy of Sciences,* **383**:44-68). MAbs 2101, 2103 and 2104 are IgM, and the remainder are IgG. Based on the Western blots, the molecular weights of the different proteins recognized by these MAbs vary between 20 and 120 kDa. (Table II). MAbs 2103, 2104, 2140 were not suitable for Western blot analysis. Results of immunoblotting and cross-competition assays suggest that MAbs pairs 2100/2101 and 2115/2116 recognize the same antigens. All other antibodies recognize distinct antigenic determinants.

In order to further characterize the antigens targeted by the different MAbs, FACS analysis was performed with various normal and tumor-derived cell lines, using different anti-BUD/RED MAbs generated. As expected, the BUD (Figure 2A1) and RED cells are positive for all the MAbs generated (Table II). Immunocytochemistry confirmed that the staining is cell surface in nature (Figure 2B). All antibodies selected also bind, to some degree, to the NODD cell line derived from adult non-obese diabetic (NOD) mouse pancreatic ductal epithelial cells and to the normal neonatal lung epithelial line, BR516 (Roberts, P. E., et al. (1992) *Animal Cell Technology: Basic and Applied Aspects,* 335-341; Roberts, P. E., et al. (1990) *Am. J. Physiol.,* **3**:L415-L425). The three antibodies which bound to the TR-1 rat endothelial cell line also bind to the other cell types tested, except cardiomyocytes (Table II, Figure 2A3, A4). These results are consistent with the immunocytochemistry results on the TR-1 cells (Figure 2C).

In contrast, MAbs 2100/01, 2103, 2104, 2160, and 2161 are more specific, and do not bind to most of the other cell types tested, including several insulinoma and acinar tumor-derived cell lines (RIN-M, RIN-F, ARIP) (Table II, figure 2A).

### Immunolocalization of Ag 2101, PDX1 and Ag 2160 along the gut at the early stage of pancreatic development

Mabs directed to BUD and RED cells were also employed to perfom immunohitochemical studies with embryonic rat pancreas. Staining of an e 12 rat embryo with MAb2100, revealed the pancreatic specificity of antigen 2101 at this stage (Figure 3A). Only sections across this region of the gut presented a strong and specific staining on the pancreatic bud. The non-specific signal visualized in the anal region was present in the controls without first antibody or with mouse isotype IgG. The immunoreativity along e12.5 rat embryonic gut was also studied using MAb 2160 (Figure 3C) and compare to the staining visualized using a rabbit polyclonal anti-rat PDX1 (Figure 3B). PDX1 immunoreactivity was seen mainly in the dorsal pancreas and at the level of a restricted area along the gut. A weaker signal was also observed in the ventral pancreas. The MAb 2160 was strongly reactive along a ventral layer of cells from the inferior part of the stomach to the ventral evagination of the pancreas. An intense signal was also visualized along the developing ducts in the dorsal pancreas and, to a lesser extend, in the ventral pancretic bud.

### Additional immunohistochemistry (IHC) studies

To better characterize the expression of various antigens during embryonic development, IHC analyses of e9 to e 18 day rat embryos, and adult pancreas, using anti-BUD/RED MAbs were carried out. Based on these analyses, the MAbs raised against the pancreatic epithelial cell lines can be roughly divided into two groups. One group, including MAbs 2100/01, 2103, 2104, 2160, and 2161, specifically targets at epithelial cells of the gastrointestinal tract and other endodermally derived epithelia (e.g. lung and kidney, whereas the other group, including MAbs 2115/16, 2117 and 2140, binds to endothelial cells and neuronal cells in addition to the aforementioned epithelical cells (data not shown). Interestingly, while the different anti-BUD/RED MAbs stained similar organs, for example the vibrissa and the rectum, the cell type stained within an organ was, in many cases, quite different. This is particularly well illustrated by comparing the staining of the vibrissa by MAbs 2117, 2160, 2161, and 2115 (Figure 4).

In the e9 rat embryo (Figure 5 A), the protein recognized by MAb 2160 is clearly present in a layer of cells corresponding to the visceral endoderm. A weak staining at the level of the extra-embryonic endoderm was also observed. In the e10 rat embryo (Figure 5 B), visceral and parietal endoderm were stained. At day e12, e15, and e18 (Figure 5 C-E) of development, epithelia of multiple organs were also stained. The MAb 2160 was strongly reactive with epithelial cells in the olfactory sinus, the lung, the intestine and the colon at e12. These structures were still positive at e15 and e18. At e15, a positive signal was also detected in the epithelium of the developing ear and pancreas. The stratified epithelium covering the olfactory sinus, oral cavity, tongue, pharynx, and trachea showed moderate to strong staining in the el8 embryo (Figure 5E). The submandibular gland and thymus were also stained. Weak to moderate immunoreactivity was observed in the lung, liver and kidney epithelium. The epithelial lining of the small and large intestine, as well as the epithelium of the urinary bladder and urethra, were strongly reactive. At 18-20 days a clear staining was observed on the membrane of the epithelial cells in the ear (Figure 5 F), the vibrissa (Figures 4 B) and in the anal canal including the rectum (Figure 5 G). Very intense to moderate staining was detected in the ductal epithelium of developing, as well as adult, pancreas (Figure 5 H). Acinar cells of the adult pancreas exihibited little or no staining and no specific signal was observed in the islets of adult pancreas except for a few cells at the periphery of the islets (Figure 5 H). No staining was observed in the muscular, skeletal or nervous tissues at any age studied.

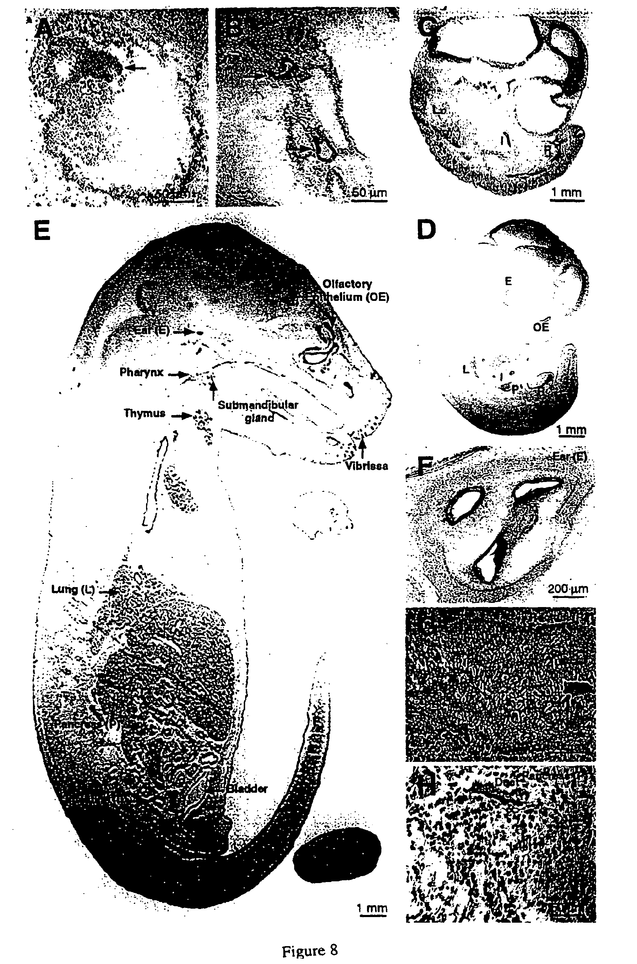

The expression of the antigen recognized by Mab 2117 has also been examined in detail. No staining was detected in e9 rat embryo itself. However, the protein recognized by MAb 2117 is abundantly expressed in the uterine endometrial lining (Figure 8A). A very intense or moderate staining was also observed in large nerve fibers in the uterine mesentery and small nerve fibers around smaller arterioles, respectively (data not shown). In e10 rat embryo, a moderate staining was observed in the notochord and no staining was evidence in the floor plate (Figure 8B 1-2). A weak staining was detectable in the uterine mesothelium. In the later development stage, the notochorde appeared intensively stained in e11 rat embryo and the floor plate moderately stained (Figure 8C). In addition, a moderate staining was observed in a dispersed population of cells lateral to the dorsal, anterior neural tube. MAb 2117 was strongly reactive with cells in the heart, lung and notochorde (Figure 8D). In e12 (Figure 8E) and e18 rat embryo (Figure 8F), Ag 2117 is more broadly distributed and is present in neuronal structure such as the spinal cord, dorsal root ganglia, some structures in the brain, nerve in the tongue, pancreas and other organs, but also in epithelial cells in the olphactory epithelium, pharynx, trachea, submandibular gland, thymus, lung, pancreas, bladder and rectum. Staining is also observed in the heart and the liver.

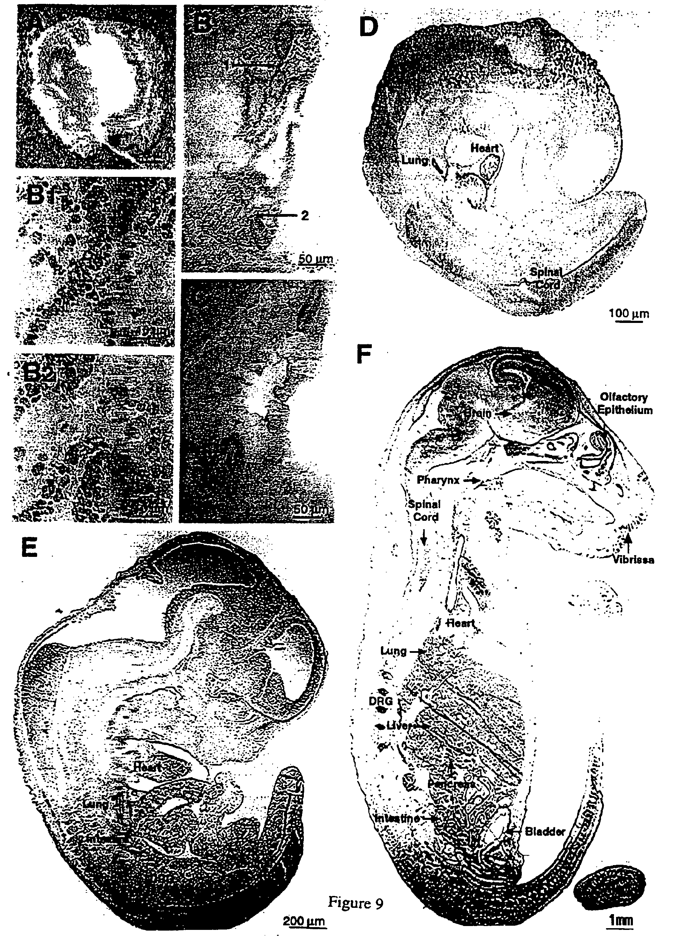

In the adult rat, MAb 2117 staining was observed in the brain, the pancreas and the kidney (Figure 9). Within the brain, an intense staining was observed in the caudate-putamen, along with fibers of the stria terminalis (Figure 9A2), in the subfornical organ and subcommisural organ (Figure 9A1, 3) and in the pia mater (Figure 9A4). No staining was observed in the ventricular epithelium. In the pancreas, a strong staining was detected for Ag 2117 in the epithelial cells of duct and acini. The MAb 2117 was also strongly reactive with nerves in the pancreas and no signal was detected in the islet (Figure 9B). In the kidney, there was an intense signal in the urinary epithelium of the renal pelvis (Figure 9C1). The juxtaglomerular apparatus appeared also positive, specifically the extraglomerular mesangial cells which were intensively stained (Figure 9C2). In the kidney cortex, the distal convoluted tubules reacted with Mab 2117.

### Cloning the genes coding for the antigens

Since all of the MAbs recognized the native configuration of cell surface antigens, the antibodies were employed in an expression cloning procedure to isolate the genes coding for the surface antigens. A cDNA library was prepared from BUD cells, and expressed in COS cells. The antibodies were then used to "pan" for cells expressing the cell surface molecules of interest. Individual clones were obtained after 5-8 rounds of panning, which started with a pool of 10 antibodies, followed by the use of individual antibodies by the the third round.

Using the high-efficiency COS cell expression system, we have purified, sequenced, and expressed the cDNA clone encoding the antigen recognized by MAb 2160 and Mab 2117. COS cells over-expressing the genes coding for antigens 2160 (Ag 2160) and 2117 (Ag 2117) showed a high level of binding of the corresponding Mabs when analyzed by FACS. No specific binding to the mock transfected COS cells was observed.

### cDNA sequences encoding Ag 2160 and Ag 2117

The DNA sequence encoding Ag 2160, shown in Figure 6A, predicted an open reading frame of 315 amino acids, with a molecular weight of 35 kDa, which is in accordance with the estimated molecular weight (Table IIA). The hydrophobicity plot of the predicted protein suggests an integral membrane protein (Figure 6B). A putative signal sequence of 11 hydrophobic amino acids is found in the sequence core. If the signal peptidase cleavage site is before the Glu-Lys-Asp sequence (von Heijne, 1986), the extracellular domain of Ag 2160 would contain 243 amino acids. The cysteine-rich extracellular domain of the protein contains two potential N-linked glycosylation sites (NXT/S) at asparagine 111 and 198, which may explain the broad band, between 40 and 50 kDa, detected by Western blotting. Ag 2160 is anchored to the membrane by a hydrophobic 23-amino acid sequence that separates the extracellular domain from a highly charged 26-residue cytoplasmic domain.

The antigen recognized by the MAb 2160 is homologous to mouse (mEGP) and human pan-epithelial glycoproteins (hEGP-1, hEGP-2). A comparison of the amino acid sequences reveals approximately 93% homology with mEGP at the amino acid level and 88% homology at the nucleic acid level (Bergsagel, P. L., et al. (1992) *J. Immunol.,* **148**:590-596). Similarly, the Ag2160 amino acid sequence shares about 88% homology with hEGP-2 (Strnad, J., et al. (1989) *Cancer Res.,* **49**:314-317) and 63% homology with hEGP-1 at the amino acid level (Linnenbach, A. J., et al. (1989) *Proc. Natl. Acad. Sc. USA,* **86**:27-31). The highest homology between Ag 2160 and EGPs is in the regions of the 12 cysteine residues, the two potential N-linked glycosylation sites, the signal and transmembrane sequences (Figure 6C).

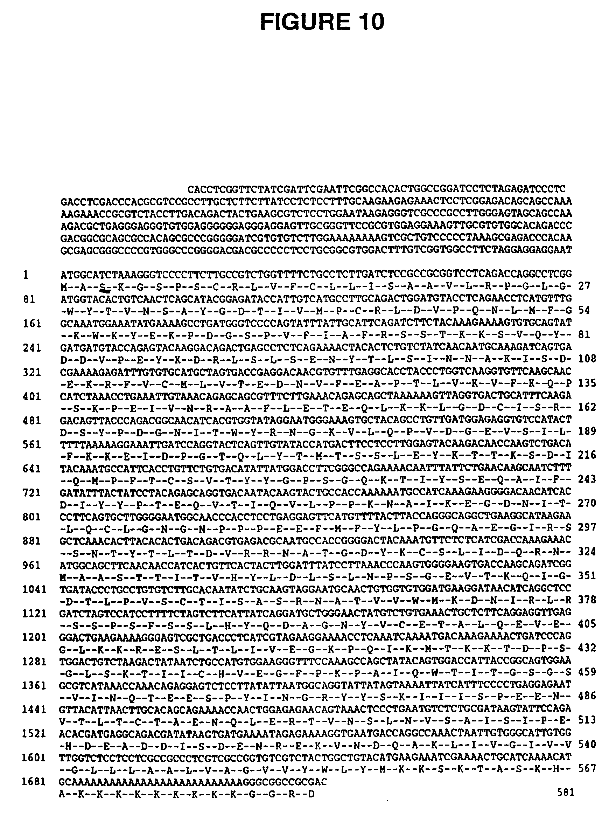

A partial cDNA clone of 2125 bp was purified using the MAb 2117 in the "panning" protocol (Figure 10). The predicted sequence of Ag 2117 is about 100% homologous to rat hematopoietic antigen (HCA), which is the rat homologue of the chicken neural adhesion molecule BEN/SC-1/DM-GRASP. The predicted amino acid sequence of the rat homologue of chicken BEN reveals the presence of several glycosylation site which probably account for the difference between the predicted molecular mass 65 kD and the 97 kD molecular mass observed on Western blot using MAb 2117 (Figure 10D).

### Tissue distribution of Ag 2160 and Ag 2117 mRNA in human

The expression of Ag 2160 mRNA was analyzed in various normal human adult tissues by Northern blotting, using the full length cDNA clones 2160. Expression of a 1.7 kb Ag 2160 mRNA was detected in the pancreas, kidney, lung, small intestine, colon, thyroid, and, to a lesser extent, in the stomach and trachea (Figure 6D). This is in good agreement with the distribution of the antigen seen with IHC in the rat embryo. No signal was detected in the muscular, skeletal and nervous tissues.

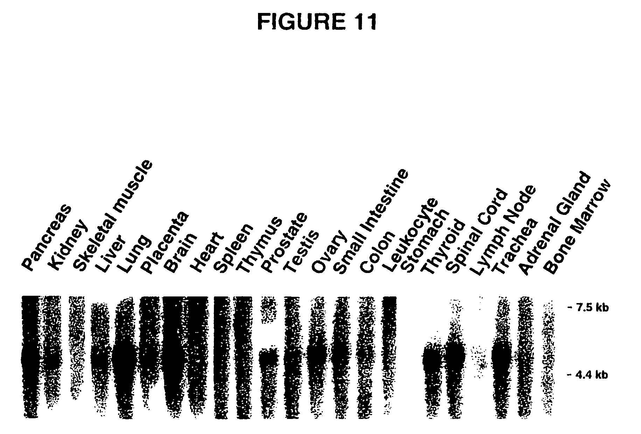

Northern blot analysis was also performed using a probe comprising the full-length 2117 cDNA. In normal human tissues, a high to moderate level of expression of Ag2117 mRNA (5 kb) was detected in the pancreas, kidney, liver, lung, placenta, spleen, prostate, ovary, small intestine, colon, stomach, thyroid, and trachea, as well as in the tissues of the nervous system, including brain and spinal cord (Figure 11). These results are in accordance with the results of immunohistochemistry analysis performed with rat embryo sections and rat adult tissue sections. In addition, no signal was detected in the human heart for Ag 2117 mRNA, suggesting that the expression observed in the embryonic heart could be transient.

### Biological activities of MAb 2160 and the fusion protein P2160

In order to understand the biological role of Ag 2160, we constructed, expressed and partially purified, a fusion protein linking the Ag 2160 extracellular domain to an HIS-6 tag (called P2160). We then examined the *in vitro* and *in vivo* biological activities of MAbs 2160 and P2160. To test the *in vitro* effect of these reagents on cells expressing the corresponding antigens, BUD cells were cultured in the presence of increasing concentrations of MAbs 2160, P2160, or of a non-relevant control antibody or control HIS-tagged protein. After 5 days of culture, cells were trypsinized and cell number and volume determined. As shown in Figure 7, treatment with Mab216 cells resulted in a dose dependent inhibition of BUD cell growth, as well as a dose dependent increase in the cell volume. The inhibitory effect of MAb 2160 was observed when as little as 1 µg/ml (6.25 nM) Mab2160 was used. The maximal effect of MAb 2160 was defected when 10 µg/mls of antibody was applied, i.e., 33% growth inhibition and 12% increase of the BUD cell volume. The MAb 2160 had no effect on TR-1 cell growth or volume, consistent with the lack of MAb 2160 binding in the FACS analysis. In addition, culture of BUD cells for 5 days in the presence of concentrations of up to 100 µg/ml of non-relevant control antibody had no effect on either cell growth or cell volume.

Similarly, BUD cells were plated in the presence of increasing concentrations of P2160. Culture of the BUD cells in presence of P2160, also resulted in a dose dependent inhibition of cell growth. The minimal concentration of P2160 required to inhibit growth was 1 µg/ml (28.6 nM). A dramatic inhibition (~> 70%) of the proliferation of the cells and an increase in cell volume (~7%) were observed when 100 µg/ml of P2160 was added to the culture media. Similar to the effect mediated by MAb 2160, increased cell volume correlated with the decrease in cell number following the treatment with P2160. BUD cell number or volume was unaffected by the addition of the control HIS-6 fusion protein (Figure 7B).

Considering the effect of MAb 2160 and P2160 on BUD cell growth and volume, it seemed possible that Ag 2160 might signal through changes in protein phosphorylation of the cytoplasmic domain of the protein and/or other associated cytoplasmic proteins. To determine the influence of treatment of BUD cells either with MAb 2160 or P2160 on phosphorylation status, confluent cell cultures were lysed, immunoprecipitated either with anti-phosho-Ser/Thr/Tyr MAb or with MAb 2160, separated by gel electrophoresis, transferred and immunobloted with anti-phosho-Ser/Thr/Tyr MAb. As shown in Figure 6C, a 2 hr treatment of the cells with MAb 2160 resulted in the appearance of a 50-kD phosphorylated protein. The phosphorylation of this protein occurs on a tyrosine, since the corresponding band is also present when the membrane was probed with an anti-phosphotyrosine MAb. No significant change was seen in the phophorylation levels when the cells were treated for 2 hr with the fusion protein P2160, after immunoprecipitation of the phosphorylated proteins. However, the appearance of a 100-kD phosphorylated protein and a decrease of the phosphorylation of a 28-kD protein, were observed when P2160 treated cell lysate was immunoprecipitated with MAb 2160. Parallel immunolabelling with specific anti-P-tyr suggests that the 100-kD protein was phosphorylated on a serine or a threonine and the 28-kD protein on a tyrosine. In addition, the immunoprecipitated Ag 2160 itself appears to be phosphorylated on a tyrosine.

In sum, we have established two cell lines from the early stages of pancreatic differentiation. These cell lines express marker proteins including cytokeratin 7, PDX1, carboxypeptidase A, tyrosine hydroxylase that are characteristic of embryonic pancreatic epithelial cells. A large body of evidence suggests that these early epithelial cells eventually give rise to the ductal, islet and acinar cells in the adult pancreas (Debas et al. (1997) *Am. J. Surg.* **174:**227-231). These cell lines established in serum-free media allowed us to raise antibodies that specifically recognize pancreatic epithelial cells of e12.5 embryos and epithelia of developmentally related organs. Using this strategy, we generated more than 15 MAbs, including the 10 presented here, which were specific for cell surface proteins, with minimal cross-reactivity to embryologically unrelated cells, e.g., mesodermally derived tissues. All of the MAbs that we raised using this method recognize the extracellular domain of surface antigens. Furthermore, these Mabs preferably bind to antigens exhibiting their native configuration, as those being preserved in the frozen tissues and sections thereof. Many Mabs we generated do no recognize denatured antigens immobilized on a Western blot.

### Example 11 Comparative analysis of various cell immunogens

By way of illustration, this set of experiments demonstrates that viable and intact cells cultured in serum-free medium are better immunogens than cells cultured in serum-supplemented medium in generating monoclonal antibodies capable of binding to cell surface antigens representative of the type of cells used for immunization.

In this study, adult Schwann cells (rASCs) derived from rat dorsal root ganglia were employed as the immunogens. rASCs were isolated and propagated in serum-free medium according to the procedures detailed in U.S. Patent No. 5,721,139, U.S. Patent No. 5,714,385, and Li, R. (1997) *Endocrinology,* **138**:2648-2657. Balb/c mice were immunized either intraperitonealy (ip) or via the foot pad (fp) with approximately 5 X 10⁶ to 5 X 10⁷ intact rASC cells grown in serum-free or serum-supplemented medium.

After several rounds of boost immunization, hybridomas were generated by fusing lymphocytes from the mouse spleen (for the group of mice being inoculated interperitonealy) or the mouse lymph nodes (for the group of mice being inoculated at the foot pad) with the myeloma line X63-Ag8.653 using about 35% to about 50% polyethylene glycol 4000 (Oi, V. and Herzenberg, L. (1980) "Immunoglobulin-Producing Hybrid Cell Lines"). An approximately equal number of hybridoma clones was obtained from the two sub-groups of mice being immunized intraperitonealy with rASC cells grown in medium supplemented with or without serum. However, four times more positive hybridoma clones were found to produce monoclonal antibodies directed to surface antigens of rASC cells when serum-free cells were employed as immunogens. As shown in Table 1, whereas only 3% of the hybridomas generated from the mice immunized with serum-cultured rASC cells secrete monoclonal antibodies reactive with intact rASC cells, 12% of the hybridomas obtained from the mice injected with serum-free rASC cells produce monoclonal antibodies exhibiting specific binding to intact rASC cells as determined by FACS analysis (see Example 3 for detailed experimental procedures concerning FACS). Similarly, a two-fold difference in the percentage of positive hybridoma clones was observed in the group of mice received foot pad immunization with the same immunogens (Table 1). Clearly, cells whose surfaces are free of serum are more effective in generating monoclonal antibodies capable of binding to surface antigens, regardless of the route of inoculation.

**TABLE 1:**

| Condition | Intraperitoneal immunization with rASC cells cultured in medium supplemented with 10% fetal calf serum (without adj uvant) | Intraperitoneal immunization with rASC cells cultured in serum-free medium (without adjuvant) | Foot pad immunization with rASC cells cultured in medium supplemented with 10% fetal calf serum (without adjuvant) | Foot pad immunization with rASC cells cultured in serum-free medium (without adjuvant) |
|---|---|---|---|---|
| | | | | |
| Total No. of Hybridoma Clones | 460 | 480 | 64 | 34 |
| No. of Negative Hybridoma Clones | 448 | 422 | 63 | 33 |
| No. of Positive Hybridoma Clones | 12 | 58 | 1 | 1 |
| Percentage of Positive Hybridoma Clones | 3% | 12% | 1.5% | 3% |

| | | | | |
|---|---|---|---|---|
| The total number of hybridoma clones represents the sum of hybridoma clones generated by fusing lymphocytes with myeloma cells X63-Ag8.653. Hybridoma clones that secrete monoclonal antibodies capable of binding to intact, unfixed rASC cells as determined by a FACS analysis are denoted "positive hybridoma clones". Conversely, those hybridomas that fail to produce antibodies or that secrete monoclonal antibodies incapable of binding to intact rASC cells in a FACS analysis are categorized as "negative hybridoma clones". | | | | |

### Example 12 Effect of adjuvants in generating monoclonal antibodies reactive with cell surface antigens

We also examined the effect of adjuvants in production of monoclonal antibodies directed to cell surface antigens. Three groups of mice were employed in this study, each being immunized with adult rat Schwann cells (rASCs), adult human Schwann cells (hASCs), or embryonic human Schwann (hESCs) cells, with or without the adjuvant Ribi. Like the previous experiments shown in Example 10, these Schwann cell lines were established according to the methods disclosed in U.S. Patent No. 5,721,139, U.S. Patent No. 5,714,385, and Li, R. (1997) *Endocrinology,* **138**:2648-2657. Hybridoma production and selection were also performed as described above. For all three groups of mice, we observed several fold increase in the total number of hybridoma clones obtained by fusing the lymphocytes of the mice subjected to cell immunogens mixed with Ribi. We further noted a ten-fold increase in the percentage of positive hybridoma clones that produce monoclonal antibodies reactive with intact Schwann cells used for immunization. Thus, the use of adjuvant such as Ribi greatly enhances the yield of monoclonal antibodies directed to cell surface antigens.

**TABLE 2:**

| Cell | rASC cells injected without Ribi | rASC cells injected with Ribi | hASC cells injected without Ribi | hASC cells injected with Ribi | hESC cells injected without Ribi | hESC cells injected with Ribi |
|---|---|---|---|---|---|---|
| | | | | | | |
| Total No. of Hybridoma Clones | 34 | 187 | 29 | 72 | 85 | 216 |
| No. of Negative Hybridoma Clones | 33 | 110 | 29 | 65 | 84 | 194 |
| No. of Positive Hybridoma Clones | 1 | 77 | 0 | 7 | 1 | 22 |
| Percentage of Positive Hybridoma Clones | 3% | 41% | 0% | 10% | 1% | 10% |

| | | | | | | |
|---|---|---|---|---|---|---|
| The total number of hybridoma clones represents the sum of hybridoma clones generated by fusing lymphocytes with myeloma cells X63-Ag8.653. Hybridoma clones that secrete monoclonal antibodies capable of binding to intact, unfixed cell immunogens as determined by a FACS analysis are denoted "positive hybridoma clones". Conversely, those hybridomas that fail to produce antibodies or that secrete monoclonal antibodies incapable of binding to intact cells used in immunization are categorized as "negative hybridoma clones". | | | | | | |

## Claims

1. Use of viable and intact cells of a specific cell type, wherein the surfaces of the cells are free of serum and the cells are not human embryonic cells for immunizing a non-human host mammal to produce a population of monoclonal antibodies that bind to antigens representative of said cell type that are heterologous to the non-human host mammal, said immunizing comprising introducing into the mammal a plurality of said cells without adjuvant or with Ribi adjuvant.

2. The use of claim 1, wherein the cells have been cultured in a serum-free medium.

3. The use of claim 1 or 2, wherein the cells have been grown in the form of a monolayer.

4. The use of claim 1 or 2, wherein the cells have been grown in the form of aggregates.

5. The use of claim 1 or 2, wherein the cells have been grown on a biological or a non-biological substrate.

6. The use of claim 5, wherein the biological substrate is selected from collagen, fibronectin, laminin and poly-lysine.

7. The use of claim 5, wherein the non-biological substrate is selected from nitrocellulose, nylon and polytetrafluoroethylene membrane.

8. The use of any one of the preceding claims, wherein the cells are of fetal or adult origin.

9. The use of any one of the preceding claims, wherein the cells are of ectodermal, endodermal or mesodermal origin.

10. The use of any one of claims 1 to 7, wherein the cells are selected from the group consisting of ASC, ESC, ROG, BUD, RED, NODD, BR516, RL-65 and NEP cells.

11. A method of generating monoclonal antibodies binding to surface antigens of a specific cell type, comprising the steps of:
(a) fusing an immortalized cell line with lymphoid cells which have been taken from a mammal which has been immunized with a plurality of viable and intact cells of a specific cell type that are heterologous to the host mammal, wherein the surfaces of the cells were free of serum and the cells were introduced into the mammal without adjuvant or with Ribi adjuvant, to produce hybridomas that secrete monoclonal antibodies;
(b) culturing the hybridomas under the conditions favourable for the secretion of monoclonal antibodies; and
(c) selecting the hybridomas that secrete monoclonal antibodies binding to surface antigens present on the viable and intact cells of step (a).

12. The method of generating monoclonal antibodies of claim 11, wherein the selection is effected by an immunoassay.

13. The method of generating monoclonal antibodies of claim 12, wherein the immunoassay is selected from ELISA and immunoblotting.

14. The method of generating monoclonal antibodies of claim 11, wherein the selection is effected by a cell sorting process.

15. The method of generating monoclonal antibodies of claim 14, wherein the cell sorting process is FACS.

16. The method of generating monoclonal antibodies of any one of claims 11 to 15, wherein the surface antigens have an extracellular domain and the monoclonal antibodies bind to the extracellular domain of the surface antigens.

17. The method of generating monoclonal antibodies of any one of claims 11 to 16, wherein the cells have been cultured in a serum-free medium.

18. The method of generating monoclonal antibodies of any one of claims 11 to 17, wherein the cells have been grown in the form of a monolayer.

19. The method of generating monoclonal antibodies of any one of claims 11 to 17, wherein the cells have been grown in the form of aggregates.

20. The method of generating monoclonal antibodies of any one of claims 11 to 17, wherein the cells have been grown on a biological or a non-biological substrate.

21. The method of generating monoclonal antibodies of claim 20, wherein the biological substrate is selected from collagen, fibronectin, laminin and poly-lysine.

22. The method of generating monoclonal antibodies of claim 20, wherein the non-biological substrate is selected from nitrocellulose, nylon, and polytetrafluoroethylene membrane.

23. The method of generating monoclonal antibodies of claim 11, wherein the cells are of embryonic or adult origin.

24. A method of determining the combination of cell surface antigens present on a specific cell type, comprising the steps of:
(a) fusing an immortalized cell line with lymphoid cells which have been taken from a mammal which has been immunised with a plurality of viable and intact cells of a specific cell type that is heterologous to the host mammal, wherein the surface of the cells were free of serum and the cells were introduced into the mammal without adjuvant or with Ribi adjuvant, to produce hybridomas that secrete monoclonal antibodies;
(b) culturing the hybridomas under the conditions favorable for the secretion of monoclonal antibodies;
(c) selecting the hybridomas that secrete monoclonal antibodies binding to the cell surface antigens present on the viable and intact cells of step (a); and
(d) identifying the antigens to which the monoclonal antibodies bind, and thereby determining the combination of cell surface antigens present on said specific cell type.

25. The method of claim 24, wherein identifying the antigen of step (d) further comprises obtaining cDNAs of the specific cell type, expressing the cDNAs in a second cell type at a level of at least 5 fold higher than that of the corresponding endogenous antigens, if present, and screening cells of the second cell type for a specific binding to the monoclonal antibodies secreted by the hybridomas identified in (c).

## Patentansprüche

1. Verwendung von lebensfähigen und intakten Zellen eines spezifischen Zelltyps, wobei die Oberflächen der Zellen serumfrei sind und es sich bei den Zellen nicht um Humanembryozellen handelt, zur Immunisierung eines nichthumanen Wirtssäugetiers zur Herstellung einer Population von monoklonalen Antikörpern, die an repräsentativ Antigene für diesen Zelltyp binden, die heterolog zu dem nichthumanen Wirtssäugetier sind, wobei die Immunisierung die Einführung einer Mehrzahl dieser Zellen ohne Adjuvanz oder mit Ribi-Adjuvanz umfasst.

2. Verwendung gemäß Anspruch 1, wobei die Zellen in einem serumfreien Medium in Kultur gehalten wurden.

3. Verwendung gemäß Anspruch 1 oder 2, wobei die Zellen in Form einer Einzelschicht gezüchtet wurden.

4. Verwendung gemäß Anspruch 1 oder 2, wobei die Zellen in Form von Aggregaten gezüchtet wurden.

5. Verwendung gemäß Anspruch 1 oder 2, wobei die Zellen auf einem biologischen oder einem nicht-biologischen Substrat gezüchtet wurden.

6. Verwendung gemäß Anspruch 5, wobei das biologische Substrat aus Kollagen, Fibronektin, Laminin und Polylysin ausgewählt wird.

7. Verwendung gemäß Anspruch 5, wobei das nicht-biologische Substrat aus Nitrozellulose-, Nylon- und Polytetrafluorethylenmembran ausgewählt wird.

8. Verwendung gemäß einem der vorhergehenden Ansprüche, wobei die Zellen fötaler oder adulter Herkunft sind.

9. Verwendung gemäß einem der vorhergehenden Ansprüche, wobei die Zellen ektodermaler, endodermaler oder mesodermaler Herkunft sind.

10. Verwendung gemäß einem der Ansprüche 1 bis 7, wobei die Zellen aus der Gruppe bestehend aus ASC-, ESC-, ROG-, BUD-, RED-, NODD-, BR516-, RL-65- und NEP-Zellen ausgewählt sind.

11. Verfahren zur Herstellung von monoklonalen Antikörpern, die an Oberflächenantigene eines spezifischen Zelltyps binden, das die Schritte umfasst:
(a) Verschmelzung einer immortalisierten Zelllinie mit lymphoiden Zellen, die einem Säugetier entnommen wurden, das mit einer Mehrzahl von lebensfähigen und intakten Zellen eines spezifischen Zelltyps, die zum Wirtssäugetier heterolog sind, immunisiert wurde, wobei die Oberflächen der Zellen serumfrei waren und die Zellen ohne Adjuvanz oder mit Ribi-Adjuvanz in das Säugetier eingeführt wurden, zur Herstellung von Hybridomas, die monoklonale Antikörper sezernieren,
(b) in Kultur halten der Hybridomas unter den günstigen Bedingungen für die Sekretion von monoklonalen Antikörpern, und
(c) Selektion derjenigen Hybridomas, die monoklonale Antikörper sezernieren, die an Oberflächenantigene binden, die auf den lebensfähigen und intakten Zellen aus Schritt (a) vorhanden sind.

12. Verfahren zur Herstellung von monoklonalen Antikörpern gemäß Anspruch 11, wobei die Selektion durch einen Immunassay durchgeführt wird.

13. Verfahren zur Herstellung von monoklonalen Antikörpern gemäß Anspruch 12, wobei der Immunassay aus ELISA und Immunblotting ausgewählt wird.

14. Verfahren zur Herstellung von monoklonalen Antikörpern gemäß Anspruch 11, wobei die Selektion durch einen Zellsortierprozess durchgeführt wird.

15. Verfahren zur Herstellung von monoklonalen Antikörpern gemäß Anspruch 14, wobei es sich bei dem Zellsortierprozess um FACS handelt.

16. Verfahren zur Herstellung von monoklonalen Antikörpern gemäß einem der Ansprüche 11 bis 15, wobei die Oberflächenantigene eine extrazelluläre Domäne besitzen und die monoklonalen Antikörper an die extrazelluläre Domäne der Oberflächenantigene binden.

17. Verfahren zur Herstellung von monoklonalen Antikörpern gemäß einem der Ansprüche 11 bis 16, wobei die Zellen in einem serumfreien Medium in Kultur gehalten wurden.

18. Verfahren zur Herstellung von monoklonalen Antikörpern gemäß einem der Ansprüche 11 bis 17, wobei die Zellen in Form einer Einzelschicht gezüchtet wurden.

19. Verfahren zur Herstellung von monoklonalen Antikörpern gemäß einem der Ansprüche 11 bis 17, wobei die Zellen in Form von Aggregaten gezüchtet wurden.

20. Verfahren zur Herstellung von monoklonalen Antikörpern gemäß einem der Ansprüche 11 bis 17, wobei die Zellen auf einem biologischen oder einem nicht-biologischen Substrat gezüchtet wurden.

21. Verfahren zur Herstellung von monoklonalen Antikörpern gemäß Anspruch 20, wobei das biologische Substrat aus Kollagen, Fibronektin, Laminin und Polylysin ausgewählt wird.

22. Verfahren zur Herstellung von monoklonalen Antikörpern gemäß Anspruch 20, wobei das nicht-biologische Substrat aus Nitrozellulose-, Nylon- und Polytetrafluorethylenmembran ausgewählt wird.

23. Verfahren zur Herstellung von monoklonalen Antikörpern gemäß Anspruch 11, wobei die Zellen embryonaler oder adulter Herkunft sind.

24. Verfahren zur Bestimmung der Kombination von Zelloberflächenantigenen, die auf einem spezifischen Zelltyp vorhanden sind, das die Schritte umfasst:
(a) Verschmelzung einer immortalisierten Zelllinie mit lymphoiden Zellen, die einem Säugetier entnommen wurden, das mit einer Mehrzahl von lebensfähigen und intakten Zellen eines spezifischen Zelltyps, der zum Wirtssäugetier heterolog ist, immunisiert wurde, wobei die Oberfläche der Zellen serumfrei war und die Zellen ohne Adjuvanz oder mit Ribi-Adjuvanz in das Säugetier eingeführt wurden, zur Herstellung von Hybridomas, die monoklonale Antikörper sezernieren,
(b) in Kultur halten der Hybridomas unter den günstigen Bedingungen für die Sekretion von monoklonalen Antikörpern, und
(c) Selektion derjenigen Hybridomas, die monoklonale Antikörper sezernieren, die an Oberflächenantigene binden, die auf den lebensfähigen und intakten Zellen aus Schritt (a) vorhanden sind,
und
(d) Identifikation der Antigene, an die die monoklonalen Antikörper binden und auf diese Weise Bestimmung der Kombination von Zelloberflächenantigenen, die auf Zellen des spezifischen Typs vorhanden sind.

25. Verfahren gemäß Anspruch 24, wobei die Identifikation des Antigens aus Schritt (d) darüber hinaus die Gewinnung von cDNAs des spezifischen Zelltyps, die Exprimierung der cDNAs in einem zweiten Zelltyp in einer Menge, die mindestens fünfmal größer als bei den entsprechenden endogenen Antigenen, soweit vorhanden, ist und das Screening von Zellen des zweiten Zelltyps im Hinblick auf eine spezifische Bindung an die monoklonalen Antikörper, die von den in (c) identifizierten Hybridomas sezerniert werden.

## Revendications

1. Utilisation de cellules viables et intactes d'un type cellulaire spécifique, dans laquelle les surfaces des cellules sont dépourvues de sérum et les cellules sont des cellules embryonnaires non humaines pour immuniser un mammifère hôte non humain afin de produire une population d'anticorps monoclonaux qui se lient à des antigènes représentatifs dudit type cellulaire qui sont hétérologues par rapport au mammifère hôte non humain, ladite immunisation comprenant l'introduction dans le mammifère d'une pluralité dedites cellules sans adjuvant ou avec l'adjuvant Ribi.

2. Utilisation de la revendication 1, dans laquelle les cellules ont été cultivées dans un milieu sans sérum.

3. Utilisation de la revendication 1 ou 2, dans laquelle les cellules ont été cultivées sous la forme d'une monocouche.

4. Utilisation de la revendication 1 ou 2, dans laquelle les cellules ont été cultivées sous la forme d'agrégats.

5. Utilisation de la revendication 1 ou 2, dans laquelle les cellules ont été cultivées sur un substrat biologique ou non biologique.

6. Utilisation de la revendication 5, dans laquelle le substrat biologique est choisi parmi le collagène, la fibronectine, la laminine et la polylysine.

7. Utilisation de la revendication 5, dans laquelle le substrat non biologique est choisi parmi une membrane de nitrocellulose, de nylon et de polytétrafluoroéthylène.

8. Utilisation de l'une quelconque des revendications précédentes, dans laquelle les cellules sont d'origine foetale ou adulte.

9. Utilisation de l'une quelconque des revendications précédentes, dans laquelle les cellules sont d'origine ectodermique, endodermique ou mésodermique.

10. Utilisation de l'une quelconque des revendications 1 à 7, dans laquelle les cellules sont choisies dans le groupe constitué de cellules ASC, ESC, ROG, BUD, RED, NODD, BR516, RL-65 et NEP.

11. Méthode de production d'anticorps monoclonaux se liant aux antigènes de surface d'un type cellulaire spécifique, comprenant les étapes de :
(a) fusion d'une lignée cellulaire immortalisée avec des cellules lymphoïdes qui ont été prélevées chez un mammifère qui a été immunisé avec une pluralité de cellules viables et intactes d'un type cellulaire spécifique qui sont hétérologues par rapport au mammifère hôte, dans laquelle les surfaces des cellules sont dépourvues de sérum et les cellules sont introduites dans le mammifère sans adjuvant ou avec l'adjuvant Ribi, pour produire des hybridomes qui sécrètent des anticorps monoclonaux ;
(b) culture des hybridomes dans des conditions favorables à la sécrétion d'anticorps monoclonaux ; et
(c) sélection des hybridomes qui sécrètent des anticorps monoclonaux se liant à des antigènes de surface présents sur les cellules viables et intactes de l'étape (a).

12. Méthode de production d'anticorps monoclonaux de la revendication 11, dans laquelle la sélection est effectuée par un test immunologique.

13. Méthode de production d'anticorps monoclonaux de la revendication 12, dans laquelle le test immunologique est choisi parmi un ELISA et un immunotransfert .

14. Méthode de production d'anticorps monoclonaux de la revendication 11, dans laquelle la sélection est effectuée par un processus de tri cellulaire.

15. Méthode de production d'anticorps monoclonaux de la revendication 14, dans laquelle le processus de tri cellulaire est le FACS.

16. Méthode de production d'anticorps monoclonaux de l'une quelconque des revendications 11 à 15, dans laquelle les antigènes de surface ont un domaine extracellulaire et les anticorps monoclonaux se lient au domaine extracellulaire des antigènes de surface.

17. Méthode de production d'anticorps monoclonaux de l'une quelconque des revendications 11 à 16, dans laquelle les cellules ont été cultivées dans un milieu sans sérum.

18. Méthode de production d'anticorps monoclonaux de l'une quelconque des revendications 11 à 17, dans laquelle les cellules ont été cultivées sous la forme d'une monocouche.

19. Méthode de production d'anticorps monoclonaux de l'une quelconque des revendications 11 à 17, dans laquelle les cellules ont été cultivées sous la forme d'agrégats.

20. Méthode de production d'anticorps monoclonaux de l'une quelconque des revendications 11 à 17, dans laquelle les cellules ont été cultivées sur un substrat biologique ou non biologique.

21. Méthode de production d'anticorps monoclonaux de la revendication 20, dans laquelle le substrat biologique est choisi parmi le collagène, la fibronectine, la laminine et la polylysine.

22. Méthode de production d'anticorps monoclonaux de la revendication 20, dans laquelle le substrat non biologique est choisi parmi une membrane de nitrocellulose, de nylon et de polytétrafluoroéthylène.

23. Méthode de production d'anticorps monoclonaux de la revendication 11, dans laquelle les cellules sont d'origine embryonnaire ou adulte.

24. Méthode de détermination de la combinaison des antigènes de surface cellulaire présents sur un type cellulaire spécifique, comprenant les étapes de :
(a) fusion d'une lignée cellulaire immortalisée avec des cellules lymphoïdes qui ont été prélevées chez un mammifère qui a été immunisé avec une pluralité de cellules viables et intactes d'un type cellulaire spécifique qui sont hétérologues par rapport au mammifère hôte, dans laquelle les surfaces des cellules sont dépourvues de sérum et les cellules sont introduites dans le mammifère sans adjuvant ou avec l'adjuvant Ribi, pour produire des hybridomes qui sécrètent des anticorps monoclonaux ;
(b) culture des hybridomes dans des conditions favorables à la sécrétion d'anticorps monoclonaux ;
(c) sélection des hybridomes qui sécrètent des anticorps monoclonaux se liant aux antigènes de surface présents sur les cellules viables et intactes de l'étape (a) ; et
(d) identification des antigènes auxquels se lient les anticorps monoclonaux et donc détermination de la combinaison d'antigènes de surface cellulaire présents sur ledit type cellulaire spécifique.

25. Méthode de la revendication 24, dans laquelle l'identification de l'antigène de l'étape (d) comprend en outre l'obtention d'ADNc de type cellulaire spécifique, l'expression des ADNc dans un second type cellulaire à un taux au moins 5 fois plus élevé que celui des antigènes endogènes correspondants, s'ils sont présents, et le criblage de cellules du second type de cellules pour rechercher une liaison spécifique aux anticorps monoclonaux sécrétés par les hybridomes identifiés en (c).
